# EUROPEAN PATENT APPLICATION

(11) **EP 4 834 257 A1**
(43) Date of publication of application: **04.11.2026**
(21) Application number: 25173734.2
(22) Date of filing: 30.04.2025
(51) Int. Cl.: A61B 17/34, A61M 25/00, A61B 90/00

(54) **SUPPORT STRUCTURE FOR A NEEDLE AND A CATHETER, A SYSTEM AND A TISSUE DETECTION ARRANGEMENT**

(71) Applicant: EPIndicator ApS, 4291 Ruds Vedby (DK)
(72) Inventor: Hofman Rosenkranz, Søren, 4291 Ruds Vedby (DK)
(74) Representative: Öberg & Sjöberg AB

(57) **Abstract**

A support structure that comprises:
- a needle support, arranged to mount, or support, a hollow needle, configured to allow a catheter to enter the hollow needle,
- a lock arranged to engage and lock the catheter relative to the support structure,
- wherein the support structure forms a gap, or space, between the needle support and the lock, and the gap is configured to allow the catheter to extend through the gap, and the gap is visible, or viewable, from outside the support structure.

## Description

### Technical field

The present invention relates to devices or systems for performing a medical procedure, in which a needle is inserted, subcutaneously in a patient's body.

### Background

An epidural is a procedure that involves injecting a medication into the space around the spinal nerves of a patient. The space around the spinal nerves may also be known as the epidural space. The goal of an epidural procedure is to provide pain relief (analgesia) or a complete lack of feeling (anesthesia) for one region of your body, such as your legs or belly. An epidural procedure may involve the steps of: the insertion of a hollow needle into the patient's back, until the tip of the hollow needle enters the epidural space just outside of your spinal cord. A catheter may be inserted through the hollow needle into the epidural space. The needle may be removed leaving a portion of the catheter in place in the epidural space. After the hollow needle has been removed the catheter may be used to provide anesthetic medicine through the catheter to the epidural space.

A lumbar puncture, also known as a spinal tap, is a procedure performed to extract cerebrospinal fluid from the spine, or spinal canal, of a patient. The extracted cerebrospinal fluid may be tested and used to diagnose certain health conditions. During a lumbar puncture, a hollow needle may be inserted into the space between two lumbar bones, called vertebrae. A catheter may be inserted into the hollow needle and the catheter may be used to extract a sample of cerebrospinal fluid from the patient.

A problem with performing an epidural or lumbar puncture, relates to the correct positioning of the needle inside the body of the patient. The correct placement of the needle relies on the experience of the physician performing the procedures. For example, when performing an epidural, the incorrect placement of the needle and thereby also the catheter may result in that no pain relief is achieved by the patient. In addition, pushing the needle into and past the epidural space may result in unwanted bleeding or leakage of cerebrospinal fluid of the patient. This in turn may result in complications and extended time of convalescence.

One method of determining the position of the hollow needle in the patient is the "loss of resistance" method. During the loss of resistance method the needle is connected to a syringe. The barrel of the syringe may hold a fluid, such as air or a saline solution. The plunger is pressed to apply a compressive force on the fluid in the barrel. As the needle traverse tissue in the patient, the tissue acts as a blockage to the needle tip, and the fluid in the barrel is prevented from exiting the syringe via the needle tip. The pressure applied by the physician may be felt as a constant force. When the needle tip enters the epidural space, fluid can exit the barrel via the needle tip, and a sudden loss of pressure, or change in force acting on the plunger, may be felt by the physician. This gives the physician physical feedback of the position of the needle tip. A problem with the method is that the fluid may be pushed into the tissue or epidural cavity of the patient. This is undesirable. The method further requires extensive training and thereby also experience of the physician performing the method.

### Object

It is thereby an object of the present inventive concept to provide a device and a system that reduces the risk that the needle or catheter is incorrectly placed in a patient. It is also an object to improve the probability that an unexperienced physician correctly performs the procedure with lowered risks for the patient.

### Summary

The object is attained by means of a support structure and a system, having the features defined in the independent claims. Preferred embodiments of the proposed technology are further defined in the dependent claims.

According to a first aspect of the proposed technology, a support structure is proposed that comprises:
- a needle support, arranged to mount, or support, a hollow needle, configured to allow a catheter to enter the hollow needle,
- a lock arranged to engage and lock the catheter relative to the support structure,
- wherein the support structure forms a gap, or space, between the needle interface and the lock, and the gap is configured to allow the catheter to extend through the gap, and the gap is visible, or viewable, from outside the support structure.

According to a second aspect of the proposed technology, a system is proposed that comprises:
- the support structure according to the first aspect,
- a hollow needle and a catheter,
- wherein the hollow needle is mounted to the needle support,
- wherein the lock, locks the catheter relative to the support structure, and
- the catheter extends from the lock, through the gap, or space, enters the needle at the needle support, extends through the needle and beyond a needle tip, and
- wherein at a movement of the catheter relative to the needle support, or the needle tip, the catheter deforms, or buckles, in the gap, or space, and the deformation is visible, or viewable, from outside the support structure.

The gap, or space, may permit the state of the catheter to be viewed, or observed, by the physician. In addition, the gap, may permit the deformation of the catheter at the movement of the catheter relative to the needle support, or the needle tip. In extension, the physician may determine the position of the hollow needle in the patient, including if the needle tip is positioned in tissue or the epidural space. Visual feedback may be more distinct, and easier to apprehend, compared to present procedures based on loss of pressure that primarily relies on providing tactile feedback to the physician.

It is understood that the epidural space is the area, or volume, or space, between the dura mater, the outermost layer of the spinal meninges, and the inner surface of the vertebral canal. It is understood that as the needle enters the patient the needle may pass through skin, subcutaneous fat, back muscles, supraspinous ligament, interspinous ligament, ligamentum flavum and into the epidural space. During a lumbar puncture the needle may further pass through dura mater and arachnoid and into the subarachnoid space. The present invention is not limited to epidural procedures or lumbar punctures. The present invention may be used to perform procedures wherein the catheter is to be positioned in a cavity, or space, in the patient. It is further understood that the cavity, or space, may be an air-filled cavity or a fluid filled cavity.

It is specified above that the gap is configured to allow the catheter to extend through the gap, and the gap is visible, or viewable, from outside the support structure. Thereby the deformation of the catheter is visible, or viewable, or observable, from outside the support structure. It is understood that the deformation of the catheter, or buckling, or bending, is related to the movement of the catheter relative to the needle support, or the needle. Worded differently, when the catheter moves in the hollow needle, or the catheter tip is pushed into the hollow needle at the needle tip, the catheter is deformed in the gap, and the deformation is visible, or viewable, from outside the support structure. It is understood that when the catheter deforms in the gap, the tip of the catheter may be aligned with the needle tip. The deformation of the catheter in the gap may thereby be at a movement of the catheter relative to the needle support, or the needle tip. The gap is thereby configured to, or arranged to, or shaped to, permit deformation of the catheter in the gap.

It is specified above that the support structure comprises, or has, a needle support, arranged to mount, or support, a hollow needle. The support structure may have a proximal end, or first end, and a distal end, or second end. The first end of the support structure may comprise the needle support. When the support structure together with a needle mounted to the needle support is used to insert the needle into a patient, the first end, may face the patient, or the portion of skin, the needle is inserted into. It is understood that the first end of the support structure faces in an opposite direction from the second end, or distal end. Worded differently, the first end is opposite the second end. The first end and the second end may be separated by the gap. This means that the gap of the support structure is arranged between the first end and the second end of the support structure. The support structure may have a third end, or a lower end, or a bottom end, and an opposite fourth end, or upper end. The lower end may be located opposite the upper end.

It is specified above that the support structure forms a gap, or space, between the needle support and the lock, and the gap is configured to allow the catheter to extend through the gap. Worded differently, the gap, or space, may form an opening in the support structure, that is configured to allow the catheter to extend through, or across, the opening. The gap may form a cavity in the support structure.

The gap may be configured to allow the catheter to extend across the gap. This means that the catheter can enter, extend across, or over, the gap, and exit the gap. The gap is thereby configured to receive a catheter, and the catheter may extend through the gap.

The gap may be defined by at least one continuous wall that encloses the gap. For example, the wall may be a continuous wall that encircles the gap. The continuous wall may outline a circle, or an ellipse. The continuous wall may be formed in the upper end of the support structure. Worded differently, the upper end of the support structure may define an upper surface, and the continuous wall may form a recess that forms part of the upper surface. This means that the recess may be formed in the upper surface.

Alternatively, the gap may be defined by a first wall and a second wall, wherein the first wall may be spaced apart from the second wall. Worded differently, the support structure may have a first wall and the second wall, that together define the gap. The gap may have a length, and the length of the gap may be defined by the first wall and the second wall. Worded differently, the first wall may be located at a distance from the second wall, and the gap may be located, between the first wall and the second wall. The first wall may be located at the proximal end, or first end, of the support structure, and the second wall may be located at the distal end, or second end, of the support structure.

The first wall may be a straight wall. The second wall may be a straight wall. It is understood that a straight wall may be free from curves, bends, angles, or irregularities. Alternatively, the first wall may be a curved wall, such as convex or concave. The second wall may be a curved wall, or convex or concave. The first wall and the second wall may be parallel. Worded differently, the first wall and the second wall may be abreast. In one example, the first wall and the second wall may be concave, and the first wall and the second wall may be connected. For example, the first wall and the second wall, may together form the continuous wall, discussed above. In a further example, the first wall may form a half-circle and the second wall may form a half-circle, and the first wall may be connected to the second wall, and together form the continuous wall that outlines a circle.

The first wall may define a first plane, and the second wall may define a second plane. The first plane and the second plane may intersect. Worded differently, the first wall defined by the first plane and the second wall defined by the second plane may form an angle relative to each other.

The gap may extend through the support structure. Worded differently, the gap may form an opening in the support structure, and the opening may extend from the third end to the fourth end of the support structure. This means that the gap may form openings at both the third end and the fourth end of the support structure.

Alternatively, the gap may have, or define, a bottom wall, or bottom. This means that the gap does not extend through the support structure, from the third end to the fourth end of the support structure. The bottom wall may define a plane. The bottom wall may limit deformation of the catheter in the gap. Worded differently, the gap may be defined by the first wall, the second wall and the bottom wall, and the gap may permit deformation of the catheter in the gap. this means that deformation of the catheter is in the gap, primarily parallel to, or in, the plane defined by the bottom wall.

The first wall and a second wall may be connected to the bottom wall. Worded differently, the bottom wall may connect the first wall to the second wall. The first wall, the second wall and the bottom wall may form the recess in the support structure. It is understood that the recess may define, or outline, the gap.

It is specified above that the gap may be defined by the first wall and the second wall, wherein the first wall is spaced apart from the second wall. It is also specified above that the first wall and the second wall may be concave, and the first wall and the second wall may be connected. The first wall may have, or define, a first height, or wall height. Worded differently, the first wall may define a first depth, or wall depth, of the gap. The second wall may have, or define, a second height, or wall height. Worded differently, the second wall may define a second depth, or wall depth, of the gap. The first height may be the same as the second height. The depth of the gap may then constant, not vary between the first wall and the second wall. Alternatively, the first height may be different from the second height. The depth may then vary between the first wall and the second wall. The first wall may be connected to the second wall via a third wall. Worded differently, the first wall may be connected to the third wall and the second wall may be connected to the third wall. The third wall may be transverse to the first wall or the second wall. The first wall may be connected to the second wall, and the first wall and the second wall may further be connected to the third wall. This means that the gap may be defined by the first wall, the second wall and the third wall.

The first wall may be connected to a fourth wall, and the second wall may be connected to the fourth wall. The first wall, the second wall, the third wall and the fourth wall may define the gap, or an opening of the gap. Each of the first wall, the second wall, the third wall and the fourth wall may be connected to the bottom wall. The fourth wall may be parallel to the third wall. The fourth wall may be transverse to the first wall and/or the second wall. The third wall and the fourth wall may each have a wall height, or wall depth.

The first wall, the second wall, the third wall, and/or the fourth wall may be connected to the bottom.

The first wall, the second wall, the third wall, the fourth wall and the bottom may form, or define, or outline, the gap. The gap may define a rectangular opening, or square opening. Alternatively, the gap may define a circular or elliptical opening.

It is specified above that the first wall may be connected to a fourth wall, and the second wall may be connected to the fourth wall. Each of the first wall, the second wall, the third wall and the fourth wall may be connected to the bottom wall.

The first wall, the second wall, the third wall and/or the fourth wall may extend vertically, or away, from the bottom wall and enclose the gap. Alternatively, the first wall, the second wall, the third wall and/ or the fourth wall may extend vertically, or away, from the support structure and enclose the gap. This means that the first wall, the second wall, the third wall and the fourth wall, each forms a protrusion that extends from the support structure, and together enclose the gap. In this example, the gap does not form a recess in the support structure. Instead, the first wall, the second wall, the third wall, and the fourth wall, or the gap, forms a protrusion that protrudes from the support structure.

It is specified above that the support structure has the needle support, arranged to mount, or support, the hollow needle, wherein the needle support is configured to allow a catheter to enter the hollow needle. The support structure may have a first opening configured to allow the catheter to enter the hollow needle via the needle support. The first opening may be configured to allow the catheter to extend from the gap into the hollow needle. Worded differently, the first opening may connect the gap to the needle support. The opening may be formed in the first wall. Worded differently, the opening may be formed at the first wall. The first opening may extend through the first wall. The opening may extend from the gap to the needle support. It is understood that the needle support may constitute the first opening, or a part of the first opening. Worded differently, the needle support may form part of the first wall, and the opening may be configured to allow the catheter to extend from the gap to the hollow needle, via the needle support.

The first opening may be defined by an axis. The axis may be transverse to the first wall. Worded differently, the axis may be transverse to the first plane of the first wall. The axis may form an angle relative to the first wall or the first plane of the first wall. The axis may be parallel to a normal axis of the first wall, or the first plane of the first wall. This means that the axis may be orthogonal to the first wall.

It is specified above that the support structure may have a first opening configured to allow the catheter to enter the hollow needle via the needle support. The support structure may have a further, or second, opening. The second opening may be arranged opposite the first opening, relative to the gap. Worded differently, the first opening may be formed in the first wall and the second opening may be formed in the second wall. This means that the first opening and the second opening may be located at opposite walls, or opposite sides, of the gap. It is specified above that the gap is configured to allow the catheter to extend through, the gap. The catheter may extend from the first opening, across, or through, the gap, to the second opening. It is specified above that the support structure comprises the lock, and that the lock is arranged to engage and lock the catheter relative to the support structure. The second opening may be arranged to permit, or allow, the catheter to extend from the gap, to the lock. The second opening may connect the gap with the lock, and the catheter may extend from the gap, through, or via, the second opening, to the lock. Worded differently, the second opening may receive and guide the catheter from the gap, to the lock.

It is understood that the lock may constitute the second opening, or a part of the second opening. This means that the lock may comprise the second opening. Worded differently, a part of the lock may form the second wall of the gap.

The second opening may be defined by an axis. The axis may be transverse to the second wall. Worded differently, the axis may be transverse to the second plane of the second wall. The axis may form an angle relative to the second wall or the second plane of the second wall. The axis may be parallel to a normal axis relative to the second wall or the second plane of the second wall.

The axis of the first opening may be a first axis, and the axis of the second opening may be a second axis. The first axis may be parallel to the second axis. The first axis and the second axis may be aligned. The first axis and the second axis may be coaxial.

Alternatively, the first axis and the second axis may be transverse. The first axis and the second axis may intersect. This means that the first axis and the second axis extend at an angle relative to each other. The first axis and the second axis may form an angle relative to each. The angle may be an obtuse angle. The angle may be 90°-180°, 120°-150° or 130°-140°.

It is specified above that the first opening may be formed in the first wall and the second opening may be formed in the second wall. Alternatively, the second opening may be formed in the third wall or the fourth wall.

It is specified above that that the support structure may have the first opening and the further, or second, opening. The first opening and/or the second opening may be an aperture, or orifice, or channel, or groove. The first opening and/or second opening may be circular, or elliptical or rectangular. The first opening and/or the second may define a closed geometrical shape, or closed-cross sectional shape, such as a circle. Alternatively, the first opening and/or the second may define an open geometrical shape, or open-cross sectional shape. Worded differently, the first opening and/or the second may form a channel, defined by a plurality of walls, that together form an open cross-sectional shape. For example, the first opening and/or the second may be a channel, that is shaped like a hyoid. Worded differently, the first opening and/or the second may define a U-shaped channel.

It is specified above that the support structure may have a first opening configured to allow the catheter to enter the hollow needle via the needle support. It is understood that a catheter has an outer diameter. It is further understood that the first opening may have, or be defined by, a width, or diameter. The width, or diameter of the first opening may be larger than the outer diameter of the catheter. This allows the catheter to enter, and extend through, the first opening. The width, or diameter, of the first opening may be a factor greater than the outer diameter of the catheter. For example, the width, or diameter, of the first opening may be 110% to 300% of the outer diameter of the catheter. Worded differently, the width, or diameter, of the first opening may be 1.1 times to 3 times the outer diameter of the catheter.

It is further understood that the second opening may have, or be defined by, a width, or diameter. The width, or diameter of the second opening may be larger than the outer diameter of the catheter. This allows the catheter to enter the second opening. The width, or diameter, of the second opening may be a factor larger than the outer diameter of the catheter. For example, the width, or diameter, of the second opening may be 110% to 300% of the outer diameter of the catheter. Worded differently, the width, or diameter, of the second opening may be 1.1 times to 3 times the outer diameter of the catheter.

The width, or diameter, of the first opening may be the same as, or equal to, the width, or diameter, of the second opening. Alternatively, the width, or diameter, of the first opening may be different from the width, or diameter, of the second opening. For example, the width, or diameter, of the second opening may be larger than the width, or diameter, of the first opening. This may make it easier for the catheter to deform, or buckle, in the gap, at the movement of the catheter relative to the needle, or needle support.

The width, or diameter, of the first opening and/or the width, or diameter, of the second opening may be constant through the longitudinal length of the respective opening. Worded differently, the first opening and/or the width, or diameter, of the second opening may be constant along the axis of the respective opening. Alternatively, the width, or diameter of the first opening and/or the width, or diameter, of the second opening may be variable, or vary along the opening. For example, at the gap, the respective opening may have a greater width, or diameter, relative to the width, or diameter, of the opening at the needle support, or at the lock.

It is specified above that the gap is configured to allow the catheter to extend through the gap. This means that a section of the catheter may extend through the gap, and occupy the gap. Worded differently, the gap may be configured to receive the section of catheter, that extends through the gap.

It is also specified that at a movement of the catheter relative to the needle support, or the needle tip, the catheter deforms, or buckles, in the gap, or space.

The catheter may thus move in the hollow needle, at least in the longitudinal extension direction of the hollow needle. The gap may be sized, or shaped, to allow the catheter to deform in the gap, and the deformation may be viewable, from outside, or external to, the support structure. Worded differently, the gap may be configured to permit the catheter to deform in the gap. The gap may be configured to allow, or permit, the catheter to deform, or buckle, within the gap, at a movement of the catheter relative to the needle support, or needle tip, or needle, at an engagement of the lock. This means that when the catheter is engaged by the lock, and the catheter moves relative to the needle support, or needle, or needle tip, the catheter may deform, or buckle, in the gap.

The movement of the catheter relative to the needle, or needle support, may be due to a force applied to the catheter tip in a lengthwise direction, or longitudinal direction, of the catheter. The lengthwise direction may also be referred to as the axial direction of the catheter. It is understood that the deformation of the catheter is an elastic deformation. Worded differently, the catheter can deform elastically in the gap. It is understood that in engineering, deformation may be elastic or plastic. It is further understood that elastic deformation is a temporary deformation, whereas plastic deformation is a permanent deformation. The deformation of the catheter being an elastic deformation means that when the force applied in the lengthwise direction of the catheter is removed, the catheter returns to the undeformed state, or the state the catheter had before the force was applied. The elastic deformation of the catheter a is thus a reversible deformation, and not an irreversible deformation as is the case for plastic deformations.

The catheter may extend through the hollow needle, and extend beyond the tip of the needle. The movement of the catheter may be in a longitudinal direction of the needle. It is understood that the movement of the catheter relative the needle support, or needle tip, is a movement that increases the length of catheter in the gap. Worded differently, at a movement of the catheter relative to the needle support, or the needle tip, the length of catheter in the gap increases. It is understood that the movement of the catheter relative to the needle support, or needle tip, is the result to a catheter lengthwise, or axial, compressive force, and not the result of a catheter lengthwise, or axial, tension force. It is further understood that a tension, or tensile, force, is a force that act to elongate or stretch an object. It is also understood that a compressive force is a force that act to compress or squeeze a material and that when a compression force is applied to the catheter, it causes the catheter to undergo deformation in the opposite direction of the compressive force.

For example, when a portion of catheter that extends beyond the tip of the needle, is pushed, or forced, into the hollow needle, the length of catheter in the gap may increase, and the catheter may deform, or buckle, in the gap.

The deformation of the catheter in the gap may be a deformation in a deformation direction. The deformation direction may be transverse to the first opening and/or the second opening.

The deformation direction may be transverse to the longitudinal direction of the needle. Worded differently, the deformation direction may be transverse to the first axis of the first opening. The deformation direction may be transverse to the second axis of the second opening. The movement of the catheter in the hollow needle may thereby result in a deformation of the catheter in the gap, wherein the deformation is transverse to the hollow needle.

The deformation direction may be transverse to both the first axis and the second axis. The deformation direction may be transverse to the first axis and parallel with the second axis. The deformation direction may be parallel with the first axis and transverse to the second axis. In a further alternative, the deformation direction may be both parallel with the first axis, and parallel with the second axis. The deformation direction may be in the first direction and in a second direction. The first direction may be the X-direction and the second direction may be the Y-direction.

The deformation of the catheter may be, or occur, between the first opening and the lock, or second opening. Worded differently, the deformation of the catheter may be largest at a point of the catheter located between the first opening and the lock, or the second opening. It is understood that the deformation primarily takes place in the gap. This means that the deformation is local to the portion of catheter in the gap. The deformation, or buckling, may form a radius bend in the catheter. It is understood that the radius may decrease at the deformation of the catheter. This means that as the deformation of the catheter in the gap increases the radius of the deformation may decrease.

It is specified above that the gap may be defined by the bottom wall, or bottom and that the bottom wall may define the plane. The deformation of the catheter may be in the plane. Worded differently, the catheter may deform and the deformation may be parallel to the bottom, or bottom wall. This means that the deformation of the catheter can easily be viewed relative to the bottom wall. The deformation of the catheter may be limited, or constrained, by the bottom wall. This means that during deformation of the catheter, the catheter deforms in a direction that is parallel to the bottom, or the plane.

It is specified above that at the movement of the catheter relative to the needle support, or the needle tip, the catheter deforms, or buckles, in the gap, or space.

The deformation, or buckling, of the catheter in the gap, may form a bulge, or bend, in the catheter, and the deformation may bend, or buckle, the catheter in the deformation direction. The bulge, or bend, in the catheter may form a smooth bulge, or bend, in the catheter. The deformation may be a Euler buckling. It is understood that a Euler buckling is a deformation wherein the catheter will deform and form a smooth bend, or buckle. It is further understood that an Euler buckling is separate from a shear crimping deformation or a wrinkling deformation, that do not form a smooth bend, or buckle, of the catheter.

It is specified above that the first wall may be connected to the second wall via the third wall. The third wall may be a continuous and straight wall. Alternatively, the third wall may form an apex. Worded differently, the third wall may be convex, or form a bulge. The apex may face the centre of the gap. The third wall may contact the catheter that extends through the gap. At least the apex may contact the catheter that extends through the gap. The third wall, or the apex, may contact and pre-bend the catheter before the catheter deforms at the movement of the catheter relative to the needle support, or needle tip. The catheter may thus have a first state and a second state. The first state may be a relaxed state, before the movement of the catheter relative to the needle, needle tip, or needle support. The second state may be a deformed state, wherein the catheter is deformed, at the movement of the catheter relative to the needle, needle tip, or needle support. It is understood that the third wall may pre-bend the catheter in the first state.

The pre-bend of the catheter may be in the deformation direction. Worded differently, the third wall may be convex, or form a bulge, that has an apex, and the apex may contact and pre-bend the catheter in a first direction. The third wall may thus be configured to contact and exert a transverse load, or biasing force, on the catheter that deforms, or pre-bends, the catheter in the first direction.

The first direction may be parallel to the deformation direction. This means that the third wall pre-bends the catheter in the deformation direction. This ensures that the catheter deforms, or buckles, in the deformation direction at the movement of the catheter relative to the needle support, or needle tip, and not in the opposite direction of the deformation direction.

Alternatively, the third wall may comprise a protrusion, that protrudes outwardly, from the third wall, in the first direction. The protrusion may form the apex of the third wall. The protrusion may contact and pre-bend the catheter in the first direction, and at the movement of the catheter relative to the needle support, or needle tip, the catheter deforms in the deformation direction, wherein the first direction is parallel to, or aligned with, the deformation direction. It is specified above that the third wall may comprise a protrusion. The third wall may comprise a plurality of protrusions, that each protrude in the first direction, each forming an apex, wherein each apex may contact the catheter, and together pre-bends the catheter in the first direction.

It is specified above that the axis of the first opening may be the first axis, and the axis of the second opening may be the second axis and that the first axis may be parallel to the second axis and that the first axis and the second axis may be aligned. The first axis and the second axis may be coincident. The first axis and the second axis may be coaxial. The apex may extend from the third wall and cross the first axis and the second axis. This means that the catheter that extends from the first opening, across the gap, to the second opening, will form a pre-bend at the apex, between the first opening and the second opening. The apex may extend beyond, or past, the first axis and the second axis by between 1-10 mm, such as between 2-5 mm. The apex may extend beyond, or past, the first axis and the second axis by at least 1 mm.

This has the effect of pre-bending the catheter in the gap, which results in that, at the movement of the catheter relative to the needle support, or needle tip, the catheter will bend in the deformation direction.

It is specified above that the gap may have a length, and the length of the gap may be defined by the first wall and the second wall. The apex may be located halfway between the first opening and the second opening. Worded differently, the apex may be located at half the length from the first wall or the second wall.

It is specified above that the first wall may be connected to the second wall via the fourth wall. The fourth wall may be a continuous and straight wall. Alternatively, the fourth wall may be concave. The fourth wall may be configured to limit the deformation of the catheter at the movement of the catheter relative to the needle, needle tip, or needle support. Worded differently, when the catheter deforms in the gap, the catheter may contact the fourth wall, and the fourth wall may act to stop further deformation of the catheter in the deformation direction. In the first state, or relaxed state, of the catheter, the catheter may contact the third wall, or the apex, of the third wall. In the second state, or deformed state, the catheter may contact the fourth wall. Worded differently, the catheter may deform in the gap, between the first state and the second state, wherein the catheter contacts the third wall in the first state and the fourth wall in the second state. The third wall and the fourth wall may define a maximum distance between the third wall and the fourth wall. The maximum distance may define the maximum deformation of the catheter in the gap. The maximum distance may be 5-20 mm. In one example, the third wall is convex and the fourth wall is concave, wherein the convex third wall matches, or corresponds to, the concave fourth wall. This means that the convex third wall may define a first radius, and the concave fourth wall may define a second radius, wherein the first radius is equal to the second radius. The third wall and the fourth wall may together limit the maximum deformation of the catheter, and prevent the catheter tip, at the needle tip, from being pushed to far into the hollow needle.

It is specified above that the third wall may comprise a protrusion, that protrudes outwardly, from the third wall, in the first direction, and that the protrusion may form the apex of the third wall. The bottom, or bottom wall, may comprise the protrusion that protrudes outwardly from the surface of the bottom, and the protrusion may comprise the apex. Worded differently, the protrusion may protrude outwardly from the bottom, and a surface of the protrusion may form the apex, that contacts pre-bends of the catheter in the first direction, or the deformation direction.

It is specified above that the gap is visible, or viewable, from outside the support structure. It is also specified above that the gap may be defined by the bottom wall, or bottom. The bottom may be visible, or viewable, from outside the support structure. This allows the deformation of the catheter in the gap, relative to the bottom, to be visible, or viewable. The bottom wall may comprise an indicia, or scale, or marking, that indicates the amount of deformation of the catheter at the movement of the catheter relative to the needle support, or needle tip. The scale may be a continuous measurement scale. Alternatively, the scale may have a mark that indicates when the catheter tip has entered the needle fully, and deformed in the gap. The indicia, or scale, may be applied to a surface of the bottom wall. For example, the indicia may be printed onto the surface. Alternatively, the indicia may be engraved or embossed in the surface. In one example, the indicia is printed onto the surface in a colour different from that of the surface.

The gap may be covered. The support structure may comprise a cover, that covers the gap. The gap may then be further defined by the cover. The cover may be parallel to the bottom. The cover may be transverse to the first wall and the second wall. The cover may be configured to allow the gap to be viewed from outside the support structure. The cover may be transparent. It is specified above that the bottom wall may limit deformation of the catheter in the gap. The cover may limit the deformation of the catheter in the gap. Worded differently, the cover may be parallel with the bottom, and the cover may define a second plane. The second plane may be parallel to the plane defined by the bottom. The two planes may then together prevent deformation of the catheter in a direction transverse to the bottom and the cover. Worded differently, the bottom and cover may be configured to, or arranged to, enable deformation of the catheter in the plane of the bottom or the plane of the cover.

It is specified above that the support structure has the needle support, arranged to mount, or support, a hollow needle, configured to allow a catheter to enter the hollow needle. The hollow needle may thus be mounted, or attached, to the needle support. The needle support may be configured to allow a catheter to enter the hollow needle. The needle support may comprise an opening, or channel, or aperture. The opening may be configured to receive and guide the catheter into the hollow needle.

The opening may be coupled to, or open to, the hollow needle. This means that a catheter may enter the hollow needle via the needle support.

The needle support may be arranged at the end of the support structure. The needle support may be located at the first wall. Worded differently, the needle support may be located at the first wall. It is understood that the needle support is arranged at the first end of the support structure. It is understood that the needle support may be located in, on, or near, the first wall. It is

The needle support may be configured to releasably attach the hollow needle. The needle support may comprise a connector that connects to the hollow needle. The connector may be a needle adapter. The connector may be configured to connect to a hub on the hollow needle. The hollow needle may thus be attached to, or mounted to, the needle support of the support structure. The needle adapter may outline a protrusion that can interface with a matching recess, or hub, of the hollow needle.

The needle support may form a protrusion that interfaces with a needle hub. The needle support may form a connector, or hub, that extends away from the distal end and detachably connects to the needle hub of a needle. It is understood that the connector is hollow.

It is specified above that the support structure may have a third end, or a lower end, and an opposite fourth end, or upper end. The bottom end may be located opposite the upper end. The bottom end may be connected to, or form, a handle. Worded differently, the handle may extend from, and be joined to, the support structure. The handle may be designed to, or configured to, be grasped by a hand of the physician. Alternatively, the handle may form a separate part, or body, that is mounted to, or joined with, or connected to, the support structure. The bottom end of the support structure may then have a connector that connects to a matching connector on the handle. The bottom of the support structure may form a groove that interfaces with a track formed on the handle. The track of the handle may then slide onto, or into, the groove of the support structure.

The handle may form a space configured to hold, or store, a portion of the catheter, such as a coil of catheter. The handle may comprise a spool, or reel, located in the space, wherein the spool is configured to hold the portion of the catheter, or a coil of the catheter. Worded differently, the space may form an opening in the handle configured to allow the coil of catheter to be inserted and removed from the space.

The handle may comprise a first side wall and a second side wall, wherein the first side wall and the second side wall are connected via a third side wall and a fourth side wall. The first side wall may further be connected via the spool, or reel. Alternatively, the first side wall and the second side wall may be configured to attach, or mount, the spool, or reel. For example, the spool, or reel, may form an axle, and the first side wall and the second side wall may have a respective slot, or groove, the axle may mount to. In a further example, the first side wall and the second side wall may each have an opening, or through hole, configured to receive and mount the axle. This means that the spool of catheter may be mounted to the handle via the slots, or grooves; or openings, or through holes, arranged on the first side wall and the second side wall.

It is specified above that the handle may form a separate part, or body, that is mounted to, or joined with, or connected to, the support structure. This means that the catheter may be pre-assembled to the handle. Before performing the procedure, the handle may be mounted to the support structure and the catheter may be extended across the gap, and enter the needle support, and the hollow needle.

It is specified above that the support structure may comprise the lock, arranged to engage and lock the catheter relative to the support structure. Worded differently, the support structure may have the lock configured to lock, or fixate, the catheter relative to the lock. When the lock engages and locks the catheter to the support structure, and at a movement of the catheter relative to the needle support, or the needle tip, the catheter may deform, or buckle, in the gap, or space. This means that, at the movement of the catheter relative to the needle support, or needle tip, the catheter is unable to move relative to the lock. This results in that the catheter deforms, or buckles, in the gap. The lock may be arranged at the second end of the support structure. Worded differently, the lock may be located at the second wall. The lock may be located in, on, or near, the second wall.

The lock may have a locked state and an unlocked state. In the locked state, the lock may engage and, and lock, the catheter relative to the support structure. In the unlocked state, the lock may disengage, and unlock, the catheter relative to the support structure. This means that in the unlocked state, the catheter may move in its axial extension direction, relative to the support structure, and in the locked state, the catheter is prevented to move in its axial extension direction, relative to the support structure. It is understood that at the transition from the locked state and the unlocked state, the lock may disengage the catheter, and at the transition from the unlocked state to the locked state, the lock may engage the catheter.

The lock may be an interference lock that engages and biases the catheter against the support structure. The lock may comprise a movable interference element, that contacts and engages the catheter. The interference element may engage and bias the catheter against the support structure. This means that he catheter is clamped between the interference element and the support structure. The interference element may be movable by an actuator, or actuator mechanism. The position of the interference element may be controllable by the actuator. The actuator may be a manual actuator. For example, the actuator may be a lever, or a wedge mechanism. Worded differently, the actuator may form a lever or a wedge that engages the interference element in the locked state, or at the transition from the unlocked state to the locked state. When the actuator is actuated, the actuator may move the interference element, to bias the catheter against the support structure.

It is specified above that the lock may have a locked state and an unlocked state. The states may be set, or operated, into the locked state. This means that the lock may maintain the locked state, once the lock has been set into the locked state, without operator input, or biasing force from the operator. In the same manner, the lock may maintain the unlocked state, once the lock has been set into the unlocked state.

Alternatively, the lock may be an operator activated lock. This means that the lock engages the catheter, or assumes the locked state, when the operator biases the lock, such as by applying a biasing force on the lock. When the operator releases the lock, and no longer applies the biasing force on the lock, the lock returns to the unlocked state. Worded differently ,the lock may be biased to assume the unlocked state.

It is specified that the lock may be an interference lock, and that the lock may comprise a movable interference element, that contacts and engages the catheter. The lock may further comprise an intermediate member, or element, arranged between the interference element and the catheter. This means that the interference element does not directly contact the catheter. Instead the interference element engages the intermediate member that in turn contacts the catheter.

The intermediate member may be of a flexible material, that elastically deforms, when the lock is in the locked state. The intermediate member may be attached to the interference element. For example, the intermediate member may be coated to, or adhered to, the interference element. The intermediate member may deform around the catheter. The intermediate member may outline a groove that conforms to the shape of the catheter. In the locked state, the intermediate member may contact both the catheter via the groove and the support structure at either side of the groove. This may reduce crushing or permanent deformation of the catheter when the lock is in the locked state. Alternatively, the intermediate member may only contact the catheter when the lock is in the locked state.

The intermediate member may be of a flexible material such as a silicone or rubber material. The flexible material may lock the catheter relative to the interference element via friction.

The support structure may comprise a matching flexible material, located at the opposite side from the intermediate member of the catheter.

Alternatively, the intermediate member may form outline a flexible tube, or tube section, with an opening configured to receive the catheter. When the flexible tube is compressed, between the support structure and the interference element, the tube contacts, and locks the catheter relative to the interference element and the support structure. The flexible tube may be attached to the support structure. Alternatively, the flexible tube may be attached to the interference element.

It is specified above that the lock may constitute the second opening, or a part of the second opening. It is also specified above that the second opening may be an aperture, or orifice, or channel, or groove. The lock may interface, or engage, the catheter in the second opening. For example, second opening may form a channel, or groove, and the interference element may interface with, or match, the channel or groove. This means that the interference element may lock the catheter relative to the support structure, at the second opening. When the second opening is a channel, or groove, the interference element may form a protrusion, that may be received by the channel or groove, to engage the catheter in the groove, or channel. The protrusion may be a ridge, or elongated protrusion, that engages a length of catheter in the channel, or groove. This has the benefit that the biasing force of the interference element is distributed along a section of the catheter, and may reduce crushing or permanent deformation of the catheter when the lock is in the locked state.

It is specified above that in the lock may be an interference lock that engages and biases the catheter against the support structure. Alternatively, the lock may engage and bias the catheter to the lock itself. Worded differently, the lock may be attached to, or mounted to, the support structure and the lock may engage and lock the catheter to the lock. This means that the lock does not engage, or bias, the catheter to the support structure. This also means that the catheter is engaged and locked to the look itself, and the lock in turn attached to, or mounted to, the lock.

It is specified above that the lock may comprise a movable interference element, that contacts and engages the catheter. The lock may comprise two, or more, interference elements, that each engages, or contacts, the catheter, when the lock is in the locked state. The lock may comprise a first interference element and a second interference element. The first interference element and the second interference element may each engage the catheter, and clamp the catheter between the first interference element and the second interference element. The first interference element may be engaged by a first actuator, or actuator mechanism, and the second interference element may be engaged by a second actuator, or actuator mechanism. Alternatively, the first interference element and the second interference element may both be actuated and moved, by a common actuator. The common actuator may be the manual actuator, or actuator mechanism, discussed above. For example, the common actuator may be the lever, or the wedge mechanism, that actuates and moves both the first interference element and the second interference element.

It is specified above that the bottom of the support structure may form a groove that interfaces with a track formed on the handle. The locking mechanism may be a separate part from the support structure or the handle. The locking mechanism may have a track that interfaces with the groove of the support structure. This allows the locking mechanism to attach to the support structure. The support structure and the locking mechanism may then be used without the handle.

It is specified above that the lock may be an interference lock, and that the lock may comprise a movable interference element, that contacts and engages the catheter in the locked state. The support structure may form a groove that interfaces with a tongue, or tracks on the movable interference element. The groove and the tongue, or tracks, may be inclined relative to each other. When the moveable interference element moves relative to the groove, or tracks, of the support structure, the interference element may contact and lock the catheter relative to the support structure.

The support structure may be of plastic, or polymer, or metal material. For example the material may be polyethylene, polypropylene, polymethyl methacrylate, polyvinyl chloride, polyamide, acrylonitrile butadiene styrene, polycarbonate or stainless steel.

It is specified above that the support structure comprises the needle support, arranged to mount, or support, a hollow needle, configured to allow the catheter to enter the hollow needle. It is also specified that system may comprise the hollow needle mounted to the needle support.

It is understood that the needle has a proximal end and a distal end, or needle tip. The needle has a cutting edge at the tip of the needle. It is further understood that the needle is hollow. The hollow needle may be configured to receive the catheter, and the catheter may extend from the proximal end, or first end of the needle, to, and beyond, the distal end, or second end, of the hollow needle.

The hollow needle may comprise a shaft and a hub. The hub may be attached, or connected, to the shaft at the proximal end. The hub and the shaft may together form the hollow needle. The second end of the shaft may comprise the needle tip. The shaft may have a longitudinal extension between the hub and the tip. The shaft may have a circular cross-sectional shape.

It is specified above that the needle is a hollow needle. The shaft may define a lumen, or hollow space, configured to receive the catheter. Worded differently, the hollow needle may have a lumen, or hollow space, configured to receive the catheter.

It is specified above that the shaft may have a longitudinal extension between the hub and the tip. This means that the shaft extends along a straight axis from the hub to the tip. Alternatively, the needle may be a curved needle, wherein the longitudinal extension of the shaft, between the hub and the tip, forms a curve. The longitudinal extension of the shaft may form a constant curvature, between the hub and the tip. This means that the curvature is constant, or has a constant radius, between the hub and the tip. Alternatively, the shaft may have a first section with a straight extension and a second curved section. For example, the second section of the shaft may be curved and form the tip of the shaft. The tip of the shaft of the needle, may be a bevelled tip. Worded differently, the tip may be bevelled. In one example, the hollow needle is a tuohy needle. It is understood that the tuohy needle may have a curve at its tip designed to cause a catheter passing through the needle's lumen, past the tip, to exit laterally at a 45 degree angle. In another example, the needle may be a Crawford Needle, Hustead needle or Weiss needle.

It is specified above that the hollow needle may comprise a shaft and a hub, and the hub may be joined with, or connected to, the shaft at the first end. Worded differently, the needle may have a hub located at the first end, or proximal end, of the needle. The hub may be configured to attach, or mount, the needle to the support structure. The hub and the needle support may together form a connection interface. The hub may have a first connector and the needle support may have a second connector, and the first connector and the second connected may together be configured to attach the hollow needle to the support structure. The connection interface between the hub and the needle support may form an interference fit connection between the hub and the needle support. It is understood that an interference fit connection, may also be referred to as a pressed fit or friction fit connection. Worded differently, the hub may be attached to, and fastened to, the needle support, and the hub may be held to the needle support, via friction forces acting on the connecting, or mating, surfaces of the hub and the needle support. The needle support may thereby be arranged to, or configured to, mount, or support, the hollow needle, to the support structure.

Alternatively, the connection interface may form a mechanical connection interface. For example, the connection interface may be formed as a threaded connection interface. The hub may have a threaded first connector and the needle support may have a threaded second connector, and the first connector and the second connected may be threaded to each other, and the first connector and the second connector may together be configured to attach the hollow needle to the support structure. It is understood that the threads of the first connector and the second connector are matching, or interfacing, threads, allowing the connectors to be threaded to each other and form the threaded connection interface. The needle support may thereby be arranged to, or configured to, mount, or support, the hollow needle. For example, the threaded first connector of the hub, and the threaded second connector of the needle support may be form a locking, or slipping, Luer taper connection interface.

The gauge of the hollow needle may be between 16-22 gauge. It is understood that gauge is measured according to the Birmingham gauge/ Birmingham wire gauge unit (usually denoted as either G or ga). The length of the needle may be between 7-15 cm.

It is specified above that the needle support, is arranged to mount, or support, the hollow needle, and that the needle support configured to allow a catheter to enter the hollow needle. It is further specified above that the hollow needle may be configured to receive a catheter, and the catheter may extend from the proximal end, or first end of the needle, to, and beyond, the distal end, or second end, of the hollow needle. It is also specified above that the shaft may define a lumen, or hollow space, configured to receive the catheter. Worded differently, the hollow needle may define a lumen, or hollow space, configured to receive the catheter. The lumen, or hollow space, of the hollow needle may have a diameter, or inner diameter. It is understood that the gauge of the needle may be selected to be used with the catheter, wherein the catheter has a complementary outer diameter to the inner diameter of the hollow needle. This reduces wrinkling deformation of the catheter inside the hollow needle, and improves accuracy in determining that the hollow needle has entered the epidural space.

It is specified above that the support structure comprises the needle support, arranged to mount, or support, the hollow needle. Alternatively, the support structure and the needle may form a unitary body. This means that the needle is permanently fixed to the of the needle support of the support structure.

It is specified above that the system comprises the hollow needle and the catheter. It is also specified above that the support structure comprises the needle support configured to allow the catheter to enter the hollow needle, and that the gap is configured to allow the catheter to extend through the gap.

It is understood that the catheter may be inserted into the body of the patient together with the hollow needle. When inserted, the catheter allows for drainage, administration of fluids or gases and access by surgical instruments.

It is understood that the catheter has a first end and a second end, and that the catheter extends between the first end and the second end. It is understood that the catheter is hollow, or defines a lumen, or hollow space, that extends from the first end to the second end of the catheter. It is further understood that the catheter may have an internal diameter and an external diameter. The internal diameter may define the lumen, or hollow, space of the catheter. The catheter may have a wall thickness. The external diameter may define the outer dimensions of the catheter. The external diameter may be the thickness added to the internal diameter. The catheter may have a length, or longitudinal extension, of 30-150 cm.

It is specified above that the needle support is configured to allow a catheter to enter the hollow needle. The catheter may extend through the needle. Worded differently, the catheter may enter the needle support, extend into the hollow needle, to the needle tip. The catheter may be movable inside relative to the hollow needle. The catheter may be movable in the hollow needle in the longitudinal extension of the needle. This means that when the catheter and hollow needle has been inserted into the body cavity, the needle alone may be removed, leaving the catheter, in the body. It is understood that at least the tip of the catheter, or a section of the catheter at the first end of the catheter, may be left in the epidural cavity during the procedure. It is further understood that during insertion of the catheter and the hollow needle into the body, the catheter can move relative to the hollow needle, and that during the movement, the tip of the catheter, or a section at the first end of the catheter, may move into, or back into, the hollow needle, and the tip of the catheter may be aligned with the tip of the hollow needle, or that the tip of the catheter coincides with the tip of the hollow needle. When the catheter tip is aligned with the needle tip during insertion into tissue of the patient, the catheter tip is protected from needle-induced cutting of the catheter. In addition, kinking of the catheter tip is prevented.

The catheter may be arranged to extend beyond, the tip of the needle, before the needle and the catheter is inserted into the body of a patient. This means that a portion of the catheter, or the catheter tip, extends beyond, or past, the needle tip. The catheter may be arranged to, or configured to, extend beyond the needle tip by for example by 2-20 mm. The catheter may thereby be configured to be pushed backwards into the needle as the distal end of the needle is inserted or pushed against tissue.

It is understood that the catheter may be a thin, flexible tube. The catheter may be made of a polymer material. For example, the catheter may be a silicone, latex, nylon or Teflon catheter.

The catheter may have a stiffness, or bending stiffness, or a resistance of against bending deformation. The stiffness of the catheter is understood as the extent to which the catheter resists deformation in response to an applied force. It is understood that the material, the wall thickness and diameter of the catheter may influence the bending stiffness of the catheter. It is understood that a smaller diameter catheter has an inherently lower stiffness and may be more flexible compared to a larger diameter catheter of the same material and wall thickness.

The flexibility of the catheter is understood as the ability to deform elastically and return to its original shape when the applied force is removed.

It is understood that the deformation of the catheter is an elastic deformation. This means that the deformation of the catheter is not a permanent, or plastic deformation.

It is specified above that the bottom wall may comprise an indicia, or scale, or marking, that indicates the amount of deformation of the catheter at the movement of the catheter relative to the needle support, or needle tip. It is understood that the catheter may be transparent. It is also specified that the catheter may be made of a polymer material. It is understood that the refractive index of air is about 1 and that the refractive index of cerebrospinal fluid is about 1.33. The material of the catheter may have a refractive index that is separate or distinct from the refractive index of air. Worded differently, the catheter may be made from a polymer material, and the polymer material may have a refractive index. The refractive index of the catheter, or catheter material, may be the same, or about the same, as the refractive index for cerebrospinal fluid. Worded differently, the catheter material may be selected to have a refractive index (Rip) that matches, or corresponds to, the refractive index of cerebrospinal fluid (CSF). This means that the visibility, or legibility, or observability, of the indicia, or scale, or marking, of the bottom wall is higher when the catheter is filled with cerebrospinal fluid. By extension, this means that when the indicia is located on the bottom wall, beneath the catheter, or when the catheter is positioned between the indicia and the eye of the physician operating the system, and the refractive index of the catheter is the same, or about the same, as the refractive index of the cerebrospinal fluid in the catheter, the indicia may be clearly viewable, or observable by the physician. This also means that when air in the catheter, the indicia is less viewable, or observable, by the physician. Worded differently, when the catheter is made from a material with a refractive index matching, or corresponding to, the refractive index of cerebrospinal fluid, the occurrence of cerebrospinal fluid in the catheter can be assessed more efficiently. The indicia together with the selection of material with a refractive index that matches, or corresponds to, the refractive index of cerebrospinal fluid further improves assessing if the hollow needle has entered the spinal canal.

It is specified above that the system comprises a hollow needle and a catheter. It is also specified above that the support structure comprises a needle support configured to allow a catheter to enter the hollow needle, and that the gap is configured to allow the catheter to extend through the gap. The catheter may be configured to, or arranged to, receive a stylet. The system may comprise the stylet. It is understood that a stylet is a thin wire, or guide wire, configured to be inserted into the catheter, and that the stylet is arranged to maintain, or increase, the rigidity of the catheter. Worded differently, the catheter and the stylet may each have a stiffness, or bending stiffness, or resistance of against bending deformation, and the stylet may be received by the catheter, and the catheter with the stylet may have a combined stiffness that is higher than that of the catheter on its own, or alone. The deformation of the catheter and stylet in the gap may thereby improve, and the deformation may be distinct, and easier observable, than the deformation when no stylet is received by the catheter. It is understood that the deformation of the catheter and stylet may be an elastic deformation. Worded differently, the catheter and the stylet may deform elastically in the gap. The deformation of the catheter and stylet in the gap may thus be an elastic deformation. This means that when the force applied in the lengthwise direction of the catheter and stylet is removed, the catheter and stylet return to the undeformed state, or the state the catheter and stylet had before the force was applied. The elastic deformation of the catheter and the stylet is thus a reversible deformation, and not an irreversible deformation as is the case for plastic deformations.

It is further understood that the stylet may maintain patency of the catheter and prevent wrinkling of the catheter. Worded differently, the stiffness of the catheter may be increased by the stiffness of the stylet itself. During insertion, the combined stiffness of the catheter and stylet may reduce coiling, deviation from an intended trajectory or failure to reach the target space in the subject. It is further understood that the stylet may act as a plug, or blockage, in the catheter, that stops cerebrospinal fluid from flowing in the catheter.

The stylet may comprise, or consist of, a stainless-steel core. The core may comprise one or a plurality of strands that form the core. For example, the stylet may comprise 9, 15, 21 or 49 wire strands. It is further understood that the core may be a coated core, wherein the core is coated with a coating material. The coating material may be nylon. The coating may be electrically insulating, or non-conductive. Worded differently, the core may comprise one or more strands that form the core and the one or more strands may be coated to form the stylet. It is specified above that the catheter and the stylet may each have a stiffness, or bending stiffness, or resistance of against bending deformation, and the stylet may be received by the catheter, and the catheter with the stylet may have a combined stiffness that is higher than that of the catheter on its own, or alone. It is understood that the stylet influences the stiffness of the stylet and in extension the stiffness of the combined catheter with the stylet received within the catheter. This means that the stylet, or the configuration of material of the strands, the diameter of the strands, the number of the strands and the coating material and thickness, may be selected to achieve a desired stiffness of the stylet.

It is specified above that the catheter may be configured to, or arranged to, receive a stylet. The stylet may have a width, or diameter, that matches, or corresponds to the diameter, or inner diameter, of the catheter. Worded differently, the stylet may match, or correspond, to the gauge of the catheter. The stylet may be received by the catheter and protrude from the first end of the catheter to the second end of the catheter.

It is specified above that the support structure may comprise a lock, arranged to engage and lock the catheter relative to the support structure. It is also specified that the catheter may be configured to, or arranged to, receive a stylet. It is understood that the stylet may thereby prevent the catheter from being crushed, or collapsed, when stylet is received by the catheter and the lock engage and lock the catheter relative to the support structure.

In a further example, the second end of the catheter may also be locked to the support structure. The second end of the catheter may be locked by the catheter lock discussed above. This means that the catheter lock, locks two separate portions of the catheter relative to the support structure. The second end of the catheter may be locked by the catheter lock.

Alternatively, the second end of the catheter may be locked to an additional lock, that is separate from the catheter lock. When the second end of the catheter is also locked relative to the support structure the risk the stylet tip is pulled, or moved, away from the first end of the catheter, or catheter tip, is reduced. This may also prevent the catheter from bending at the tip of the needle when the needle with the catheter and stylet is inserted into tissue.

It is understood that the characteristics of flexibility and stiffness inherently attributes a catheter to resist bending. The configuration wherein the catheter possesses the least amount of potential energy is wherein the catheter is straight. As such, the more the catheter is bent, the more it gains potential energy.

It is understood that the catheter is in the relaxed state when it is subjected to the least amount of bending caused by the configuration of the gap while not subjected to an axial force. Worded differently, the state of which the catheter has the least amount of potential energy is herein referred to as the relaxed state, or relaxed position, of the catheter.

It is understood that the catheter requires less axial force to further bend when it is already bent in the relaxed state as compared to a straight catheter. As such it is understood that the amount of bending of the catheter in the relaxed state may vary to modify the axial force needed to further bend the catheter when the lock is in the locked state. Worded differently, less axial force is needed to further bend a catheter that is bent more compared to another catheter that is bent less in the relaxed state when the lock is in the locked state. It is understood that a larger diameter catheter needs to be bent more compared to a smaller diameter catheter in the relaxed state to further bend when subjected to the same amount of axial force when the lock is in the locked state.

It is understood that the catheter will return to the relaxed state if the axial force is lifted, such as when the distal end of the needle enters the spinal epidural space, or the lock transitions to the open state.

The diameter of the catheter is further correlated to the flowrate of liquid through the catheter. It is understood that a catheter complementary to a needle with a gauge of 16-22, or having a diameter fitting inside the needle, is suitable for the volume of liquid administered during an epidural medicine administration.

It is specified above that the catheter may be configured to, or arranged to, receive a stylet and that the system may comprise the stylet. The stylet may form part of the system. The stylet may have a first end, or distal end, and a second end, or proximal end. It is understood that the first end of the stylet may be aligned with the first end of the catheter, when the stylet is received in the catheter. It is further understood that the second end may be aligned with the second end of the catheter. The second end of the stylet may be accessible at the support structure.

The stylet may be used to check for cerebrospinal fluid. Worded differently, the stylet may be retracted relative to the catheter. The stylet may be retracted when the lock is in the open state, wherein the lock disengages the catheter. The stylet may be retracted past the gap and/or the lock. This means that the stylet does not occupy the section of catheter in the gap.

It is specified above that the catheter may be transparent. The occurrence of cerebrospinal fluid in the catheter may then be visible, or observable, to the physician. In addition, is specified above that the catheter may have a refractive index that corresponds to, or matches, the refractive index of cerebrospinal fluid. This improves the legibility of, or observability of, occurrence of cerebrospinal fluid in the catheter.

It is specified above that the system may comprise the support structure of the first aspect, the hollow needle and a catheter. It is also specified above that the system may further comprise the stylet that is received in the catheter, wherein the stylet extends from a first end, or a tip, of the catheter, to the second end of the catheter. This means that the stylet may have a first end and a second end, and that the stylet extends between the first end and the second end. The stylet may be received in the catheter, wherein the stylet extends from a first end, or a tip, of the catheter, to a second end of the catheter, wherein the stylet is arranged to form a first electrode of a tissue detection arrangement, wherein the tissue detection arrangement is configured to detect, or determine, tissue at the tip of the catheter.

In a further aspect the tissue detection arrangement is provided. The tissue detection arrangement may comprise the stylet. Worded differently, the stylet may form part of the tissue detection arrangement. The stylet may be an electrical conducting stylet. For example, the stylet may comprise, or be made of, an electrical conducting metal. In one example, the stylet is a nylon-coated stainless steel catheter, formed by stainless steel links that are linked together. The stylet may form a measurement, or first, electrode. It is understood that an electrode is a conductor through which electricity enters or leaves an object, substance, or region. The stylet may form part of the tissue detection circuit, arranged to, or configured to, detect, or determine, the tissue at the tip of the stylet. The tissue detection arrangement may continuously determine, or detect, tissue at the tip of the stylet. For example, when the hollow needle with the catheter and the stylet received in the catheter is inserted into the tissue of a subject, the tissue at the stylet tip may be continuously determined by the tissue detection arrangement. This reduces the risk that the needle with the catheter and stylet punctures risk tissue such as blood vessels or enter tissue that may lead to complications for the subject. This approach may be beneficial in procedures where real-time confirmation of anatomical layer transition is desirable, and where visual or tactile feedback alone is insufficient, such as when the catheter deforms or reforms in the gap. Examples may include, but are not limited to, epidural access in patients with ambiguous loss-of-resistance response, thoracic catheter placement in emergency or trauma settings, and tracheostomy in obese or difficult airway patients. The combined mechanical and electrical guidance may support improved accuracy, reduce procedural time, and lower the risk of tissue injury. The tissue detection arrangement may be used with the support structure with the gap discussed above. Alternatively, the tissue detection arrangement may be a stand-alone tissue detection arrangement that may be used with a generic, or general, hollow needle, catheter and conductive stylet.

The tissue detection arrangement may be used to detect, or determine tissue as well as non-tissue as well, such as an air-filled cavity or a vascular lumen. For example, information regarding the tissue surrounding the cavity, or space, may be used to determine that the needle with the catheter and stylet is close to, or adjacent, the space. This may further be used to determine high-risk tissue, such as when the needle, catheter and stylet, is close to risk tissue such as blood vessels.

The tissue detection arrangement may thus be used to detect tissue and give an indication when the needle with the catheter and stylet is close to or has entered the cavity, or space, in various procedures in a patient. Such procedures include, but are not limited to, chest drainage by determining when the needle, catheter and stylet has entered the pleural space; tracheostomy by determining that the needle, catheter and stylet has entered the tracheal airspace; and vascular access by determining that the needle, catheter and stylet has entered vascularized tissue, or the vascular lumen, wherein blood is detected instead of tissue.

The tissue detection circuit may comprise a power source. It is understood that a power source is a source of electric power. The power source may be a DC power source, or direct current power source. In one example, the power source is an electric battery. The power source may have a positive terminal and a negative terminal. The positive terminal may be connected to the measurement electrode. The electric battery may provide electric power at 1-5V, such as 3V. The electric battery may be a rechargeable battery, or a secondary cell. The power source may be connected to, or coupled to, the stylet, and the stylet may form the measurement electrode. Worded differently, the power source may be configured to, or arranged to, be connected to and provide positive electric power to the measurement electrode, or apply a positive voltage potential on the measurement electrode.

The power source may be connected to, or coupled to, the stylet via a voltage divider. It is understood that in electronics, a voltage divider (also known as a potential divider) is a passive linear circuit that produces an output voltage (Vout) that is a fraction of its input voltage (Vin). Voltage division is the result of distributing the input voltage among the components of the divider. The voltage divider may comprise a first resistance, or resistor, and a second resistance, or resistor. The voltage divider may be a resistor voltage divider. It is understood that a resistor voltage divider may commonly be used to create reference voltages, or to reduce the magnitude of a voltage so it can be measured. Worded differently, the voltage divider may output a reference voltage, or potential, (Vref).

The voltage divider may provide continuous, DC power to the stylet. The voltage divider may provide a voltage of between 50 mV to 200 mV, more preferably more than 50mV, such as about 100 mV. The voltage divider may be connected to, or coupled to, the stylet, and be configured to provide DC power to the stylet. The power source may be coupled to, or connected to, the stylet via the voltage divider. The voltage divider may be connected to the stylet at the second end of the stylet. This means that the power source may provide electrical DC power to the stylet. Worded differently, the power source may be coupled to, or connected to, the stylet and be configured to provide DC power to the stylet for detecting tissue at the first end of the stylet.

It is specified above that the stylet may form a measurement, or first, electrode. It is also specified above that the tissue detection circuit may comprise a power source and that the positive terminal of the power source may be connected to the measurement electrode.

In electronics, a comparator is a device that compares two voltages or currents and outputs a digital signal indicating which is larger. Worded differently, the comparator may compare voltages between two inputs and outputs a signal indicative of which input is largest.

The stylet may be connected to, or coupled to, and provide a first voltage, or potential, to the comparator. The comparator may further be connected to, or coupled to, a reference, or comparison, second, reference voltage, or potential. Worded differently, the comparator may be provided with a reference voltage, (Vref). The reference voltage may be provided by the power source. Worded differently, the positive terminal of the power source may be connected to, or coupled to, and provide a reference voltage to the comparator. This means that the comparator may compare the first voltage with the reference voltage. It is specified above that the voltage divider may output a reference voltage, or potential, (Vref). The comparator may be connected to, and be provided with the reference voltage from, the voltage divider. This is advantageous in that the voltage divider is connected to, or coupled to, and provides an output voltage to the stylet and to the comparator. This eliminates measurements errors during tissue detection. The comparator may further be connected to ground. It is understood that in electrical engineering, ground or earth may be a reference point in an electrical circuit from which voltages are measured, a common return path for electric current, or a direct physical connection to the Earth. It is further specified above that the power source may have a positive terminal and a negative terminal. The negative terminal of the power source may be connected to, or coupled to, the comparator. Worded differently. The negative terminal of the power source may form a ground, or earth, for the tissue detection arrangement. This has the effect that tissue detection may be achieved using one measurement electrode, in the form of the stylet, and alleviates the need to provide a second electrode for tissue detection.

Alternatively, the tissue detection arrangement may comprise the second electrode and the second electrode may form the ground. This means that the tissue detection is performed between the first electrode and the second electrode, wherein a voltage potential is applied between the first electrode and the second electrode.

It is specified above that the catheter may be made of a polymer material. The catheter may be an electrical conductive catheter. The catheter may be made from an electrical conductive polymer material. Alternatively, the conductive polymer material may have electrical conductive elements dispersed, or distributed, in the polymer material forming the catheter. In a further example, an electrical conductor may be added to the polymer material of the catheter.

Alternatively, the catheter may be a coated catheter, wherein an electrical conductive coating is applied to the catheter. The coating is preferable added to the external surface, or body, of the catheter. In this example, the catheter may form the second electrode.

In a further alternative, the hollow needle may form the second electrode, and tissue detection may be performed, between first electrode in form of the stylet and the second electrode in the form of the hollow needle.

It is specified above that the comparator may be provided with a reference voltage, (Vref). The comparator may measure, or determine, and compare the voltage between the stylet and reference voltage. It is understood that the length of the stylet, as well as the cross-sectional area of conductive portions of the stylet are known. It is also understood that the cross-sectional area of the conductive portion of the stylet is constant, and does not change during tissue detection and that the length of the conductive sections of, or the length of stylet is constant, and doesn't change during tissue detection. It is further understood that different tissue in the human body has the following conductivity. It is understood that different tissue have different characteristic and in particular different electrical conductivity. In extension, by measuring the voltage, or potential, over the first electrode and comparing the voltage to the reference voltage by the comparator may determine the tissue at the tip of the stylet. Worded differently, the power source, the first electrode and the comparator may be used to determine the tissue at the tip of the stylet, when the hollow needle, with the stylet arranged in the catheter, and protruding from the tip of the hollow needle, is inserted into tissue of the subject. The voltage drop of the first electrode corresponds to the resistance of the tissue, at the tip of the stylet, and may be used to determine the conductivity when the length of the stylet, as well as the cross-sectional area of conductive portion of the stylet is known.

It is further understood that different tissue in the human body may have the following conductivity.

**Table 1 - Tissue and conductivity**

| Tissue | Conductivity (S/m) |
|---|---|
| Skin | 0.02-0.3 |
| Adipose tissue | 0.02-0.06 |
| Ligamentum Flavum | 0.3 |
| Dura | 0.5 |
| Blood, muscle and local anaesthetics | 0.6-1.2 |
| CSF: | 1.2-2.5 |

It is further understood that the above values relating to conductivity in different tissue may be approximate as real values may vary in different patients. For example, the ligamentum flavum conductivity, may be in a range of 0.2-0.4 S/m, such as 0.25-0.35 S/m, or about 0.3 S/m. In a further example, the dura conductivity may be in a range of 0.3-0.6 S/m, such as 0.35-0.55 S/m, or about 0.5 S/m.

The comparator may be configured to compare the voltage between the stylet to the reference voltage. The voltage of, or at, the stylet may be a comparator input voltage. The comparator may compare the input voltage to the reference voltage and provide an output, or output voltage. The output voltage may be a result of the comparison of the voltage of the first electrode, or stylet, and the reference voltage. The output voltage may be provided to an output pin, or leg, of the comparator. The comparator may be a multiple output comparator, that may provide an output voltage based on the comparison between the input voltage and the reference voltage, to one or more output pins. Worded differently, the comparator may compare the input voltage to the reference voltage, and provide an output to one of a plurality of output pins based on the comparison. For example, the comparator may be a Texas Instruments LM 339 comparator, that may have four output pins, wherein one of the pins is provided with an output voltage based one the comparison of the input voltage to the reference voltage.

The output voltage may be arranged to provide an audible or visual indication based on the comparison. For example, the visual indication may be the illumination of a light source. In one alternative, the comparator may be connected to, or coupled to, a transistor via the output pin. The output voltage from the comparator may drive, operate, the transistor. The transistor may form part of an LED drive circuit, or LED switch, and based on the output voltage provided by the comparator, the LED switch may be activated to illuminate an LED, based on the output voltage provided by the comparator.

It is specified above that the comparator may be a multiple output comparator. Each output pin, or leg, may be coupled to, or connected to, an LED switch, and each LED switch may comprise a transistor, and when the comparator provides an output voltage to the respective pin, or transistor, an LED may be illuminated, indicating the tissue contacted by the stylet tip and the catheter tip.

In one example, the comparator may be coupled to, or connected to, and configured to provide an output voltage, based on the comparison between the input voltage and the reference voltage, to illuminate a first LED, a second LED and a third LED. In a further example, the first LED may be connected to a first transistor, and the first transistor may be provided with the output voltage from the comparator, and the transistor may be configured to drive, or illuminate the first LED, when the comparator provides the output voltage to the first transistor.

The second a second LED and a third LED may each be coupled to an individual output pin of the comparator via a respective second transistor and third transistor.

The first LED may be coupled to, or connected to, the first transistor via an RC circuit. It is understood that the RC circuit may comprise a resistor and a capacitor. When the comparator provides an output to the RC circuit the first LED will blink. It is specified above that the first LED may be coupled to, or connected to, the first transistor via the RC circuit.

In a further example, the first LED may form a further function for detecting tissue. It is understood that skin, in certain situations might show high conductance. For example, before inserting the needle with the hollow needle with the catheter and stylet into the skin and tissue of a patient, the skin may be disinfected. When the stylet contacts the skin high conductance may be detected resulting in a false indication may be obtained and the first LED may illuminate falsely indicating that the stylet is in skin or adipose tissue.

It is specified above that the comparator may be coupled to, or connected to, and configured to provide an output voltage, based on the comparison between the input voltage and the reference voltage, to illuminate a first LED, a second LED and a third LED. The comparator may be configured to compare the input voltage and the reference voltage, and based on the comparison and the reference conductivity of tissue, output a voltage output to illuminate one of the first LED, the second LED and the third LED.

It is specified above that the power source may be coupled to, or connected to, the stylet via the voltage divider. The stylet may be coupled to the voltage divider via a timer, or IC timer. It is understood that the timer is an integrated circuit used in a variety of timer, delay, pulse generation. The timer may be configured to provide a frequency output to the stylet. Worded differently, the stylet may be provided with pulsed DC power. It is understood that pulsed DC power, or pulsating direct current, may be a periodic current which changes in value but never changes direction. Worded differently, the pulsed DC power does not change from positive to negative potential. In other words, the pulsed DC power is not an alternative current power, that reverses direction or potential.

The pulsed DC power may have a frequency, and the frequency may be separate, or different from, 50/60Hz, common for mains AC power. In one example, the frequency is about 80 Hz. It is advantageous when the pulsed DC power has a frequency of about 80 Hz as this allows for low-pass filtering reduce electromagnetic interference common in hospital environments.

It is understood that providing pulsed DC power to the stylet reduces polarization buildup at the first end of the stylet, or at the stylet tip. This in turn reduces measurement errors and ensures consistent tissue detection at the stylet tip.

The pulsed DC power provided by the timer may be a square wave pulsed DC power. The timer may be a 555 timer.

It is specified above that the power source may be coupled to, or connected to, the stylet via the voltage divider. It is also specified above that the stylet may be coupled to the voltage divider via a timer, or IC timer. The timer may be coupled to, or connected to, the power source and the voltage divider via a operation amplifier. The operational amplifier may be coupled, or connected, in a voltage follower mode. This ensures that the reference voltage provided by the voltage divider to the comparator is the same as the voltage provided to the stylet. In extension this reduces faults in the tissue detection.

The tissue detection arrangement may further comprise an additional timer, that is first to the stylet and to the transistor that drives the green LED. The timer may be in a monostable mode, that overrides the comparator. Worded differently, the timer may be connected to, or coupled to, the stylet and the transistor that drives the first LED. When the timer is activated the timer may output a voltage signal to the transistor that activates the transistor for a predetermined time, thereby illuminating the first LED for the predetermined time. This means that upon activation, the first LED may be made to, or forced to, illuminate for the predetermined time. This allows the needle tip, the catheter tip and the stylet tip, to enter the skin and adipose tissue before tissue detection is activated. After the predetermined time has elapsed the timer may stop outputting the voltage signal.

It is specified above that the support structure forms the gap, or space. In a further alternative, the gap of the support structure may comprise a capacitance sensor plate. The capacitance sensor plate may measure, or determine, the proximity of the stylet, received in the catheter, relative to the capacitance sensor plate. When the stylet is close to the capacitance sensor plate, the voltage over the capacitance sensor plate increases. The capacitive sensor plate may be coupled to, or connected to, the first transistor that drives the first LED via a further, or additional, RC circuit. The capacitance sensor plate and RC circuit may thereby override the output voltage of the comparator provided to the first transistor that drives the first LED. This has the effect that if the stylet is blocked by adipose tissue, and thereby is unable to sense the higher conductivity of other tissue, such as the ligamentum, then all LEDs will shut off at the deformation of the catheter in the gap. It is further understood that when the hollow needle, catheter and stylet enters the epidural space, the catheter may assume the unbent shape, or state, and the stylet will move away from the capacitance sensor plate, and the above override will may deactivate.

It is specified above that the first wall may be connected to the second wall via the third wall, and/or that the first wall may be connected to the second wall via the fourth wall. The capacitance sensor plate may form part of the third wall. Alternatively, the capacitance sensor plate may form part of the fourth wall.

It is specified above that the tissue detection arrangement may comprise a comparator, and that the comparator may be coupled to, or connected to, and configured to provide an output voltage, based on the comparison between the input voltage and the reference voltage, to illuminate a first LED, a second LED and a third LED. It is also specified above that each LED may be connected to the comparator via a respective transistor. In a further alternative, the comparator and the transistors that connect, or coupled, the first LED, the second LED, and the third LED to the comparator, may be replaced by a micro processing unit, or microprocessor. It is understood that a microprocessor is a computer processor for which the data processing logic and control is included on a single integrated circuit (IC), or a small number of ICs. The comparator may thus be replaced with a micro processing unit that compares the stylet voltage to the reference voltage, and provides and output directly to the respective first LED, second LED and third LED, that drives the respective LEDs.

The micro processing unit may run executable code, that controls the comparison of the stylet voltage and the reference voltage. When the comparator is replaced with the micro processing unit, the micro processing unit may be directly coupled to, or connected to, the power source, and form the reference voltage and provide the stylet voltage to the stylet.

The micro processing unit may further be configured to, or arranged to, communicate wirelessly with a computing device, such as a smartphone. For example, the micro processing unit may be connectable to, and provide information to, or receive information from, the computing device. In one example, the micro processing unit may be adapted to communicate with the computing device over Bluetooth, over frequencies between 2.402 and 2.480 GHz, or 2.400 and 2.4835 GHz.

It is specified above that the system, and in particular the stylet may form part of the tissue detection arrangement. The tissue detection arrangement is disclosed for use with the system according to the second aspect of the present invention.

Alternatively, according to a further aspect of the present invention, the tissue detection arrangement may be used without the support structure of the first aspect, or the system of the second aspect. Accordingly, the tissue detection arrangement may comprise a general hollow needle, conductive catheter and electrical conducting stylet and the tissue detection disclosed above. Worded differently, the system according to the further aspect may comprise the hollow needle with the catheter received by the hollow needle, wherein the tip of the catheter is aligned with the tip of the hollow needle, and the stylet received in the catheter, wherein the tip of the stylet is aligned with the catheter, wherein the stylet forms the first electrode of the tissue detection arrangement and the catheter forms the second electrode of the tissue detection arrangement. This means that the tissue detection arrangement may be used with a general hollow epidural needle, a conductive catheter and a conductive stylet. In other words, the tissue detection system is not limited to be used with the system according to the second aspect disclosed herein.

### Brief description of the drawings

A more complete understanding of the abovementioned and other features and advantages of the proposed technology will be apparent from the following detailed description of preferred embodiments in conjunction with the appended drawings, wherein:
Fig. 1 shows a side view of the system comprising the support structure, a hollow needle and a catheter;
Fig. 2 shows the system of Fig.1 with the catheter removed, and the needle separated from the support structure;
Fig. 3 shows a schematic top view of the support structure;
Fig. 4A shows a schematic top view of the support structure with the catheter in an undeformed state;
Fig. 4B shows a schematic top view of the support structure with the catheter in a deformed state;
Figs. 5A-5F shows further examples of the gap of the support structure;
Figs. 6A-6C shows further schematic views of the support structure;
Figs. 7A-7C shows one example of a locking mechanism;
Figs. 8A-8D shows further examples of the locking mechanism;
Fig. 9 shows one example of the tissue detection arrangement; and
Fig. 10 shows a further example of the tissue detection arrangement.

### Description of the drawings

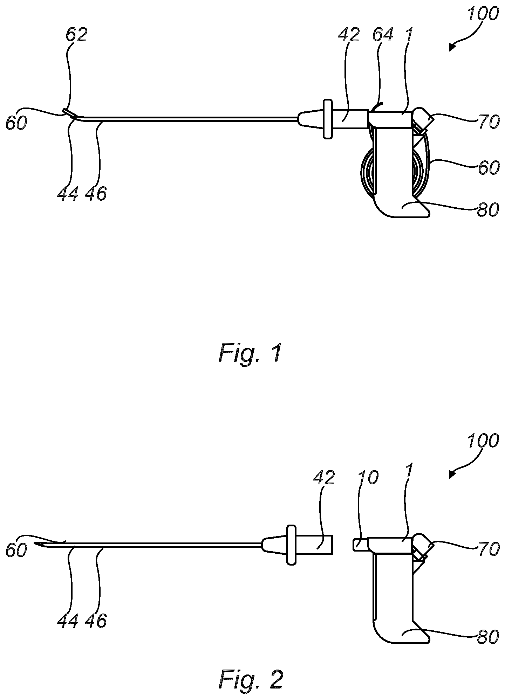

Fig. 1 shows a side view of the system comprising the support structure 1, a hollow needle 40 and a catheter 60. The hollow needle 40 is mounted to, or attached to, the support structure 1 via a needle support 10. This allows the catheter 60 to extend from the support structure 1 into the lumen, or hollow space, of the needle 40. The needle tip 44 is arranged at a distal end 52 of the needle 40. Opposite the distal end 52, or a the proximal end 50, the needle 40 has a needle hub 42. In other words, the needle 40 has a proximal end 50 and a distal end 50. The needle 40 has a cutting edge 44 at the distal end 50 of the needle 40. The distal end with the cutting edge 44 may also be referred to as the needle tip 44. The needle tip 44 is configured to be inserted into tissue. The needle 40 is configured to receive the catheter 60. The catheter 60 is movable relative to the needle tip 44. The catheter 60 can extend from, or beyond, the proximal end 50, or first end 50 of the needle 40. In other words, a portion of the catheter can extend from and beyond, the distal end 52, or second end 52, of the needle 40. Fig. 1 shows that the tip of the catheter 62, or the catheter tip 62, extends from and beyond the needle tip 44. The catheter further has a second end 64.

The needle 40 has a shaft 46 that extends between the hub 42 and the needle tip 44. The hub 42 and the shaft 46 together form the needle 40. The shaft has a longitudinal extension between the hub 42 and the tip 44. The shaft 46 of the needle 40 shown has a circular cross-sectional shape, or is a round needle.

The needle shown in Fig. 1 has a straight shaft. The needle 40 can also be a curved needle 40, wherein the longitudinal extension of the shaft, between the hub and the tip, forms a curve. The tip of the needle 40 is bevelled. A connection interface is formed between the hub 42 and the support structure 1, where the hub 42 of the needle connects to the support structure, at a first end of the support structure.

Fig. 1 further shows that the support structure has a handle attached to the support structure. The handle is configured to be held by an operator of the system 100. The handle extends from the support structure. Fig. 1 further shows that the handle forms a space configured to hold, or store, a portion of the catheter, such as a coil of catheter.

At an opposite side of the support structure, relative to the connection interface of the needle and the support structure, or at a second side of the support structure, a catheter lock is located. The catheter lock is configured to lock the catheter relative to the support structure.

The catheter of Fig. 1 extends from the lock, through the gap of the support structure, through the needle and past, or beyond the needle tip. This means that during insertion of the needle into tissue, an end, or a portion, of the catheter extends beyond the needle tip. As the catheter contacts tissue, the catheter end, or portion of the catheter that extends beyond the needle tip, is pushed into the needle. Since the catheter is locked relative to the support structure by the catheter lock, the catheter bends in the gap of the support structure, and the operator may visually assess if the needle and catheter is in tissue or has reached the epidural space.

Fig. 2 shows the system with the catheter removed and the needle removed from the support structure. Fig. 2 shows that the needle can be connected to, or attached to, the support structure 1 via the needle interface 10. At the opposite side, or the rear side, of the support structure 1, the catheter lock 70 is located.

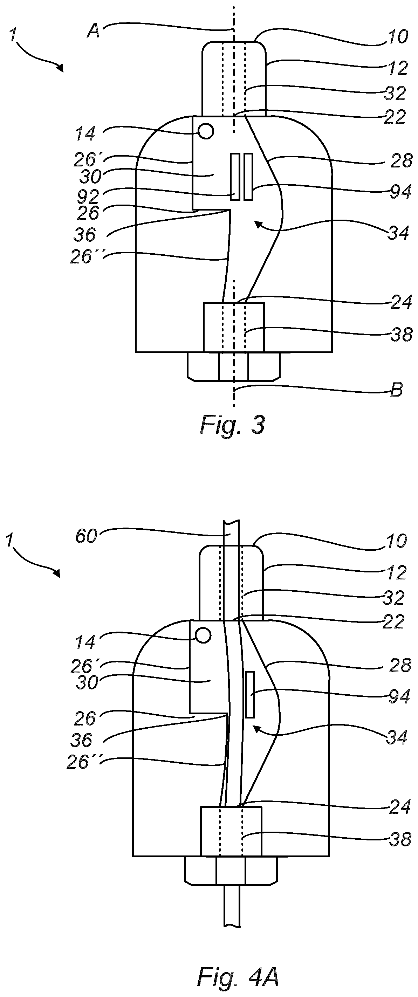

Fig. 3 shows a schematic top view of the support structure 1. Arranged at a first end of the support structure 1 is the needle support 10. The needle support 10 has a connection interface 12 configured to, or arranged to, connect to, or attach the hub 42 of the needle 40 of Fig. 1 to the support structure 1. The needle support 10 has a first opening 32 between a gap 34, or space 34, of the support structure 1 and the needle support 10 that allows a catheter to extend from the hollow needle to the gap 34. The opening 32 is shown in the form of a channel 32, that extends between the gap 34 and the tip of the needle support 10. The opening 32 is configured to, or arranged to, receive and guide a catheter from the hollow needle to the gap 34. In other words, the needle support is configured to allow the catheter to enter the hollow needle from the gap. In Fig. 3 the needle support 10 forms the first opening 32 of the support structure 1.

The gap 34 is defined by a first wall 22, a second wall 24, a third wall 26 and a fourth wall 28. The first wall 22 is located opposite the second wall 24. The third wall 26 is located opposite the fourth wall 28. The support structure 1 further has a bottom wall 30, or bottom 30. The first wall 22, the second wall 24, the third wall 26, the fourth wall 28 and the bottom 30 together define the gap. The gap 34 is configured to allow the catheter to extend through the gap 34, or across the gap 34. In other words, the gap 34 forms a cavity in the support structure 1 that allows the catheter to extend, and deform, in the gap 34.

The first wall 22 is a straight wall. The second wall 24 is a straight wall. The first wall 22 and the second wall 24 are parallel. The third wall 26 is non-straight. The third wall 26 has a first part 26' that is straight and a second part 26" that is curved. The fourth wall 28 is concave relative to a center of the gap 34.

The third wall thereby forms an apex 36. The apex 36 faces the center of the gap 34, or faces the fourth wall 28. When the catheter extends through the gap 34, the third wall 26 contacts the catheter via at least the apex 36. The catheter thereby pre-bends, or pre-deforms in the gap 34. This means that when a force is applied to move the catheter relative to the support structure 1, the catheter bends and deforms in a direction from the third wall 26 towards the fourth wall 28. The fourth wall 28 is concave and controls the maximum deformation of the catheter in the gap 34. This means that the fourth wall is configured to permit deformation of the catheter in the gap 34.

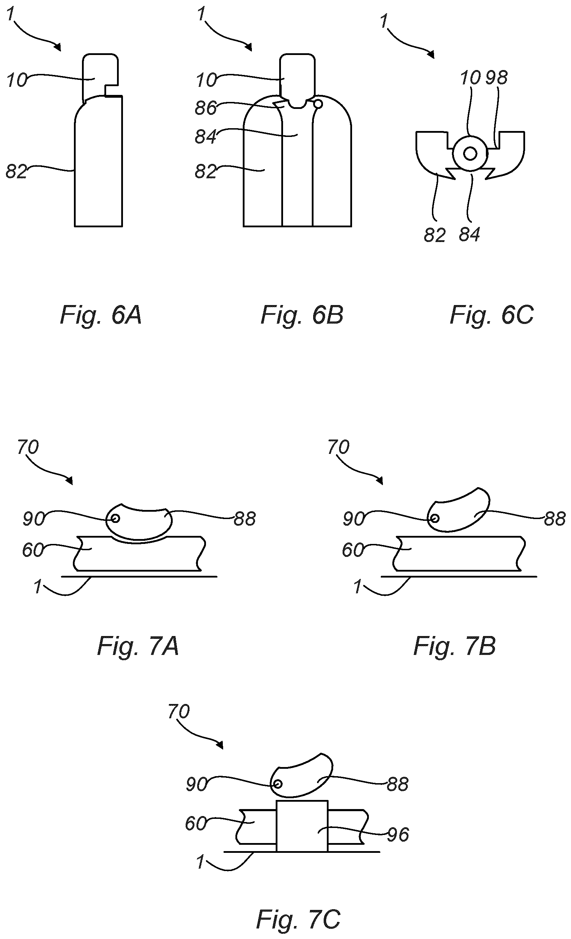

Arranged in the second wall 26 is a second opening 38. The second opening 38 is shown in the form of a channel 38, that extends between the gap 34 and the catheter lock arranged outside of the gap 34. The catheter lock, or locking mechanism, will be further discussed in relation to Figs. 7-9. The second opening 38 is configured to, or arranged to, receive and guide a catheter from the gap 34 to the catheter lock. The first opening defines a first axis "A". The second opening defines a second axis "B". As shown in Fig. 3, the first axis "A" is parallel to the second axis "B".

Fig 3 further shows a further opening 14, or catheter fixation 114, in the bottom 30 of the gap 34. The opening 14 forms part of a channel that extends from the bottom to the underside of the support structure. The channel is configured to receive and hold an end of the catheter, when the catheter is spooled or a coiled to the handle. After inserting the needle tip with the catheter into the patient, the end of the catheter is easily accessible by the operator.

The bottom wall 30 of the gap 34 comprises an indicia, in the form of two rectangular marks 92, 94. The first mark 92, and the second mark 94 indicate the amount of deformation of the catheter at the movement of the catheter relative to the needle support, or needle tip. When the catheter is aligned with the first mark 92, the catheter is undeformed which is indicative that the needle tip and catheter has entered the epidural space. When the catheter is aligned with the second mark 94, the catheter is deformed which is indicative that the needle tip and catheter is in tissue.

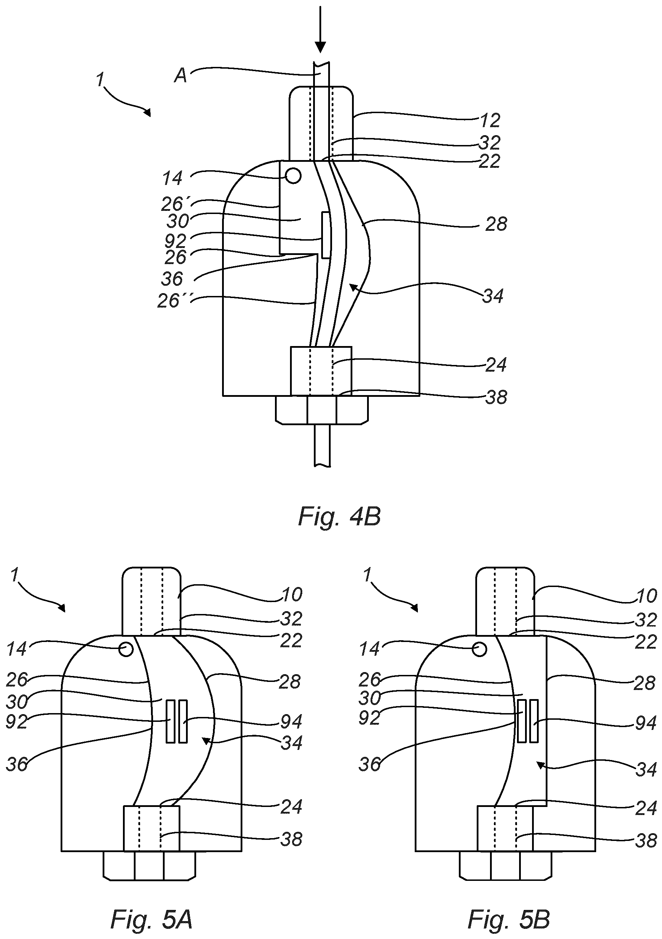

Fig. 4A shows a schematic top view of the support structure 1 with the catheter 60 in an undeformed state and Fig. 4B shows the catheter 60 in a deformed state. When the catheter is pushed into the needle, or when the catheter 60 moves relative to the needle support 10, the catheter 60 deforms, or buckles, in the gap 34, and the gap 34 together with the deformation of the catheter 60 is visible to an operator. The operator can assess if the catheter 60 has deformed in the gap 34, and can assess if the needle tip and catheter 60 is in tissue or if the needle tip and the catheter 60 tip has reached the epidural space.

Fig. 4A shows that the catheter in the undeformed state, covers the first mark, with only the second mark visible 94. This is indicative that the needle tip has entered the epidural space. Fig. 4B shows that the catheter in the deformed state covers the second mark. This is indicative that the needle tip has not entered the epidural space and is in tissue.

Figs. 5A-5F shows further examples of the gap 20 of the support structure 1, wherein Fig. 5A shows the third wall 26 being convex and the fourth wall 28 being concave. The third wall 26 has a curvature that matches the curvature of the fourth wall 28. The third wall 26 and the fourth wall 28 are abreast. The third wall 26 is configured to pre-bend the catheter in the direction towards the fourth wall 28. The third wall 26 and the fourth wall 28 together limit the deformation of the catheter 60 in the deformed state and the undeformed state.

Fig. 5B shows a further example, wherein the third wall is convex and the fourth wall is a straight wall. The third wall pre-bends the catheter and the fourth wall limits deformation of the catheter when the catheter moves relative to the needle support.

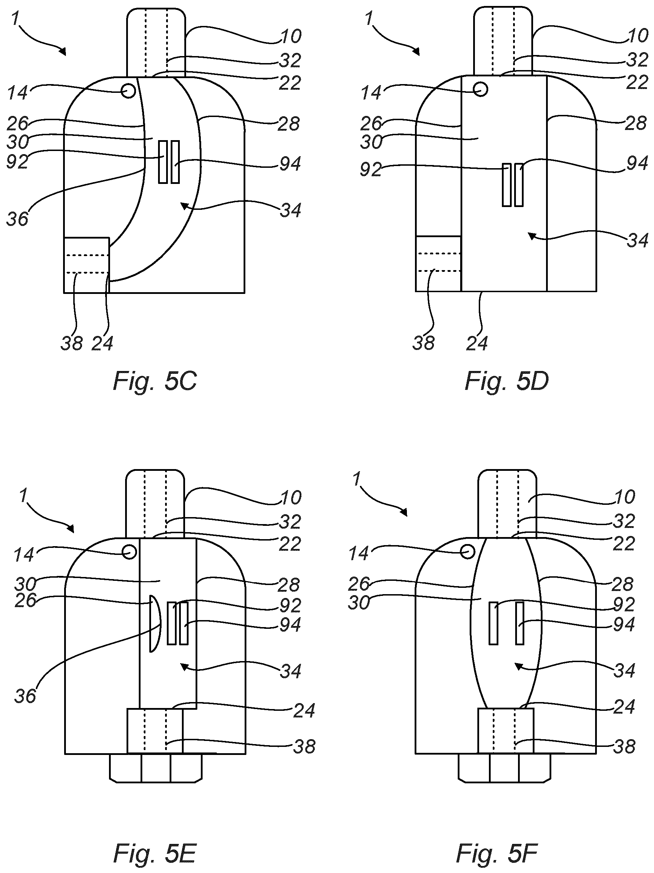

Fig. 5C shows a further example, wherein the third wall 26 and the fourth wall 28 define a curved channel that extends between the first opening 32 and the second opening 38. The second opening 38 is arranged transverse to the first opening 32.

Fig. 5D shows a further example, wherein the third wall 26 and the fourth wall 28 are both straight walls. The third wall 26 is parallel to the fourth wall 28. The first wall 22 is parallel to the second wall 24. The second opening 38 is arranged in the third wall 26. As the second opening 38 is transverse to the first opening 32, the catheter 60 will pre-bend without the need for the third wall 26 to be a curved wall, or a third wall 26 provided with an apex 36.

Fig. 5E shows a further example wherein both the third wall 26 and the fourth wall 28 are straight walls. A protrusion 48 extends from the bottom wall 30. The protrusion 48 has a curved surface facing the fourth wall 28. The curved surface of the protrusion forms the apex 36 that is configured to pre-bend the catheter 60.

Fig. 5F shows a further example of the support structure 1 with the gap 34 wherein the third wall 26 is mirrored by the fourth wall 28. The third wall 26 and the fourth wall 28 are both concave relative to a center of the gap 20. In the example of Fig. 5F the pre-bending of the catheter is not present. When the catheter 60 moves relative to the needle support 10, the catheter 60 can bend either towards the third wall 26 or towards the fourth wall 28. The first mark 92 and the second mark 94 are spaced apart to allow assessing the position of the needle tip and the catheter 60 regardless if the catheter 60 deforms towards the third wall 26 or towards the fourth wall 28.

Fig. 6A shows a side view of the support structure 1 with the handle 80 removed. Fig. 6B shows a bottom view of the support structure 1 with the handle 80 removed. Fig. 6C shows front view of the support structure 1 with the handle 80 removed. The support structure 1 has a lower end 82. The lower end 82 has a groove 84 configured to interface with a tongue arranged on the handle (not shown). This allows the support structure 1 to be slid onto the handle 80. The groove 84 has an opening 86 at the end adjacent the needle support 10 that is wider than the rest of the groove 84. The opening 84 allows the tongue of the handle to be inserted. Fig. 6C further shows that the gap 34 is covered by a transparent cover 98, or lid 98. The deformation of the catheter 60 in the gap 34 is thereby limited by the first wall 22, the second wall 24, the third wall 26, the fourth wall 28, the bottom wall 30 and the cover 60.

Figs. 7A-7B shows a first example of a catheter lock 70. The catheter lock is configured to lock, or fixate, the catheter relative to the lock. Thereby the catheter 60 is locked, or fixated, relative to the support structure 1. Fig. 7A shows the catheter lock 70 in the locked state, and Fig. 7B shows the catheter lock 70 in the unlocked state. Fig. 7A shows that in the lock 70 is an interference lock that engages and biases the catheter 60 against the support structure 1. The lock comprises a movable interference element 88, that contacts and engages the catheter 60. The movable interference element 88 of Figs. 7A-7B is pivotable around a rotation axis 90, or pivot axle 90.

Fig. 7B shows the movable interference element 88 when the lock is in the unlocked state, wherein the movable interference element 88 is not in contact with the catheter 60.

Fig. 7C shows a further example of the lock 70 comprising an intermediate member 96, or element 96, arranged between the interference element 88 and the catheter 60. The intermediate member is in the form of a tube and the catheter extends through the tube. This means that the interference element 88 does not directly contact the catheter 60 when the interference element 88 and the lock 70 is in the locked state.

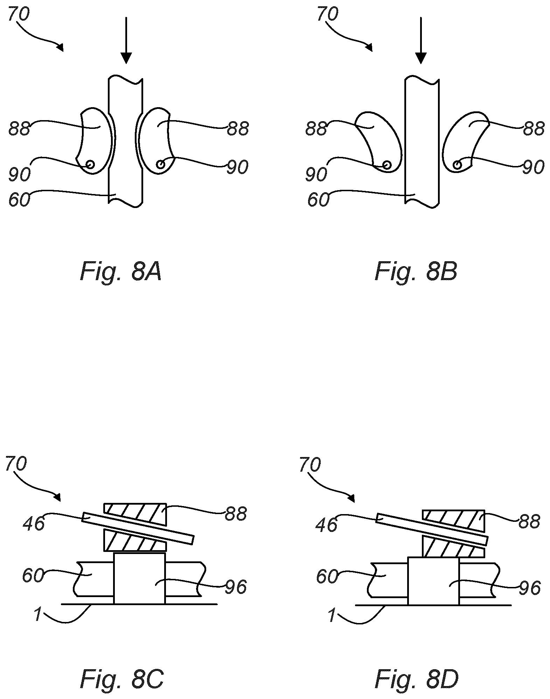

Figs. 8A-8B shows a further example of the catheter lock 70, comprising a pair of interference elements 88 arranged on opposite sides of the catheter 60. Fig. 8A shows the lock 70 in the locked state and Fig. 8B shows the lock in the unlocked state. In the locked state, the catheter is biased between the pair of interference elements 88. Figs. 8A-8B further shows that the lock shown in Figs. 8A-8B is self-locking when the catheter is pressed in a first direction.

Fig. 8C-8D shows a further example of a catheter lock in the form of a sliding wedge lock. The intermediate member 96 is in the form of a sliding wedge, that can slide along a track 46, between a first position shown in Fig. 8C, and a second position shown in Fig. 8D. Fig. 8C shows the lock in the unlocked state, and Fig. 8D shows the lock in the locked state.

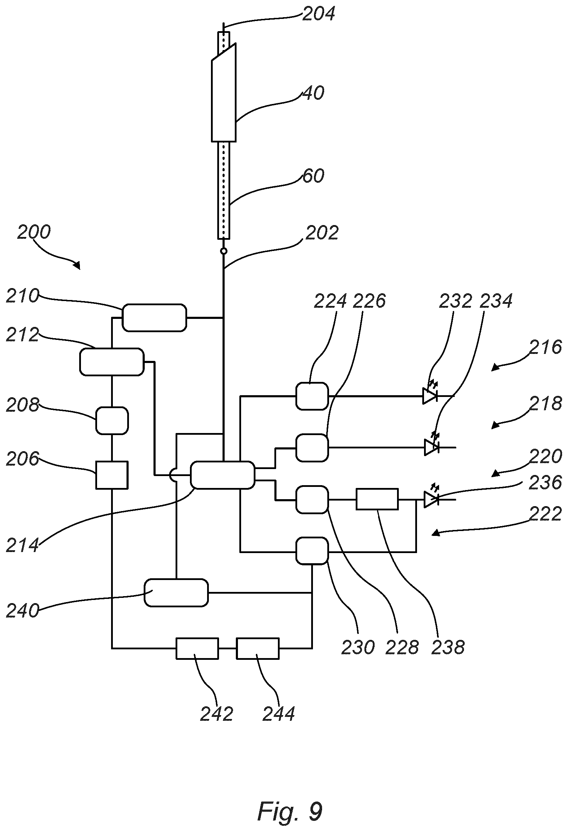

Fig. 9 shows a schematic circuitry for the tissue detection arrangement 200. The tissue detection arrangement 200 is configured for tissue detection when the hollow needle, the catheter and the stylet is inserted into tissue.

The tissue detection arrangement comprises a first conduit 202 that is configured to, or arranged to, be connected to a first electrode 204, such as a stylet 204. The stylet is an electrical conducting stylet 204. The first conduit 202 connects the stylet 204 to the tissue detection arrangement 200. The catheter 60 is received in the hollow needle 40. The stylet 204 is received in the catheter 60. The stylet 202 thereby forms the first electrode 204.

The tissue detection arrangement 200 further comprises a power source 206 in the form of a battery 206. The power source 206 provides positive DC voltage to the stylet 204, via the first conduit 202. The power source 206 is connected to, or coupled to, the stylet 204 via a voltage divider 208. The voltage divider 208 outputs a reference voltage, or potential, (Vref) to the stylet 204. Fig. 9 further shows that the voltage divider 208 is coupled to the stylet 204 via an optional timer 210, or IC timer 210. The timer 210 is configured to provide a frequency output to the first conduit 202 and the stylet 204. The stylet 204 thereby is provided with a pulsed reference voltage. The pulsed reference voltage can have a frequency that is about 80 Hz. The timer 210 is shown as a "555 timer". Fig. 9 further shows that an optional operation amplifier 212062 is connected between the voltage divider 208 and the timer 210. The timer 210 is thereby connected between the first conduit 202 and the operational amplifier 212. The operational amplifier 212 is coupled, or connected, in a voltage follower mode. The power source 206, thereby provides a voltage, or potential, to the stylet 204 via the voltage divider 208, the optional operational amplifier 212, the optional timer 210 and the first conduit 202.

Fig. 9 further shows that the tissue detection arrangement comprises a comparator 214 connected to the first conduit 202 and in extension to the stylet 204. The comparator 214 receives, or senses, the voltage of the stylet 204 and the first conduit 202.

The comparator 214 is further connected to the operational amplifier 212 and the comparator 214 receives the reference voltage as input to the comparator 214. When no operational amplifier 21 is present, the comparator 214 receives the reference voltage from the voltage divider 208 by being connected to, or coupled to, the voltage divider 208.

The comparator 214 can compare the voltage, or potential, of the first conduit 202 and stylet 204 with the reference voltage provided to the comparator 214 from the operational amplifier 212 or the voltage divider 208, in order to determine a conductivity at the tip of the stylet 204. When the tissue that contacts the tip of the stylet 204 changes, the voltage, or potential, of the first conduit 202 and stylet 204 also changes. The type of tissue that contacts the stylet 204 can thereby be determined by the comparator 214.

The comparator 214 is further connected to a first output conduit 216, a second output conduit 218, a third output conduit 220 and a fourth output conduit 222.

Based on the comparison of the comparator 214, between the voltage, or potential, of the first conduit 202 and stylet 204 with the reference voltage provided to the comparator 214, the comparator 214 provides an output to one of the first output conduit 216, second output conduit 218, third output conduit 220 and fourth output conduit 222.

Each of the first output conduit 216, the second output conduit 218 and the third output conduit 220 comprises a transistor and a visual indicator in the form of an LED.

The first output conduit 216 comprises a first transistor 224 and first LED 232, the second output conduit 218 comprises a second transistor 226 and second LED 234, the third output conduit 220 comprises a third transistor 228 and third LED 236. The comparator 214 is further connected to and provides output to a fourth output conduit 222 that comprises a fourth transistor 230. As shown, the fourth output conduit 222 does not have an LED. Instead, the fourth output conduit 222 is connected to the third LED 236 downstream the third transistor 228 of the third output conduit 220.

The transistors 224, 226, 228, 230 are shown as MOSFETs and acts as drivers for the LEDs 232, 234, 236, 238. When the comparator provides an output to one of the output conduits 216, 218, 220, 222 the respective MOSFET switches and provides power to illuminate the respective LED 232, 234, 236. The third LED 236 is further shown coupled to, or connected to, the third transistor 228 via an RC circuit 238.

Fig. 9 further shows that the fourth transistor 230 is connected to the stylet 204 via a timer 240. The timer 240 can be set to provide a voltage to illuminate the third LED 236 for a predetermined time when the tissue detection arrangement is powered on.

Fig. 9 further shows that the fourth transistor 230 is optionally connected to, or coupled to, the power source 206 via a capacitive sensor plate 242 and a further RC circuit 238. The capacitive sensor plate 242 can form part of the third wall 26 or the fourth wall 28 of the gap 20 of the support structure 1 of Figs. 3-5. The capacitive sensor plate 242 may sense when the stylet 204, and catheter 60, is close to the capacitive sensor plate 242 and activate the fourth transistor 230 to illuminate the third LED 236. When the catheter 60 with the stylet 204 extending in the catheter 60 is adjacent, or contacts, the capacitive sensor plate 242, or the wall 26, 28 the capacitive sensor plate 242 is arranged to, the fourth transistor 230 activates, or switches, to illuminate the third LED 236, in order to indicate that the catheter 60 and stylet 204 is deformed in the gap 20 of the support structure 1.

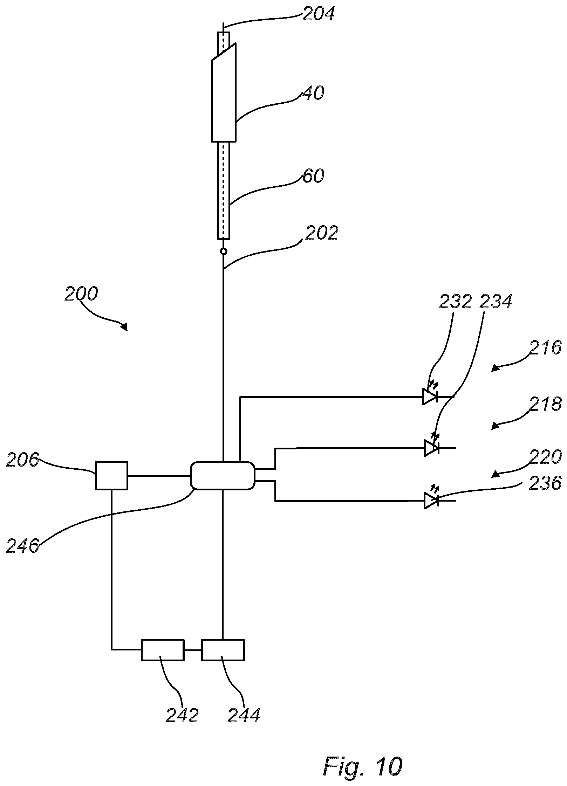

Fig. 10 shows an alternative tissue detection arrangement wherein a plurality of components of the tissue detection arrangement of Fig. 9 has been replaced with a micro processing unit 246. The power source 206 is connected to, or coupled to, the micro processing unit 246, and provides DC power to the micro processing unit 246. The micro processing unit 246, internally generates the reference voltage from the DC power received from the power source 206. The micro processing unit 246 provides a first output voltage to the stylet 204 via the first conduit 202. The micro processing unit is further directly connected to the first **LED** 232 via the first output conduit 216, the second **LED** 234 via the second output conduit 218 and to the third **LED** 236 via the third output conduit 220.

The capacitive sensor plate 242 is connected to the power source 202 and to the micro processing unit 246. The micro processing unit 246 can thus directly determine the position of the catheter 60 and stylet 204 relative to the capacitive sensor plate 242. Based on the input from the capacitive sensor plate 242 and the first conduit 202 and stylet 204, the micro processing unit can determine the tissue at the tip of the stylet 204 and provide output voltage to the first output conduit 216, the second output conduit 218 and the third output conduit 220, that selectively illuminates the respective LED 232, 234, 236 based on the sensed tissue.

### Item list

- 1: support structure
- 10: needle support
- 12: connection interface
- 14: further opening, catheter fixation
- 20: gap
- 22: first wall
- 24: second wall
- 26: third wall
- 28: fourth wall
- 30: bottom wall
- 32: first opening
- 34: gap, or space
- 36: apex
- 38: second opening
- 40: hollow needle
- 42: hub
- 44: needle tip, cutting edge
- 46: needle shaft
- 48: track
- 50: proximal end, or first end
- 52: distal end, or second end
- 60: catheter
- 62: catheter tip, first end
- 64: second end
- 70: catheter lock
- 80: handle
- 82: lower end
- 84: groove
- 86: opening
- 88: interference element, cam, sliding wedge
- 90: rotation axis
- 92: first mark, indicia
- 94: second mark, indicia
- 96: intermediate member, or element
- 98: cover
- 100: system
- A: first axis
- B: second axis
- 200: tissue detection arrangement
- 202: first conduit
- 204: first electrode, stylet
- 206: power source, battery
- 208: voltage divider
- 210: timer
- 212: operational amplifier
- 214: comparator
- 216: first output conduit
- 218: second output conduit
- 220: third output conduit
- 222: fourth output conduit
- 224: first transistor
- 226: second transistor
- 228: third transistor
- 230: fourth transistor
- 232: first LED
- 234: second LED
- 236: third LED
- 238: RC circuit
- 240: timer
- 242: capacitive sensor plate
- 244: RC circuit
- 246: micro processing unit

## Claims

1. A support structure (1) that comprises:
- a needle support (10), arranged to mount, or support, a hollow needle (40), configured to allow a catheter (60) to enter the hollow needle (40),
- a lock (70) arranged to engage and lock the catheter (60) relative to the support structure (1),
- wherein the support structure (1) forms a gap (34), or space (34), between the needle support (10) and the lock (70), and the gap is configured to allow the catheter to extend through the gap, and the gap is visible, or viewable, from outside the support structure.

2. The support structure of claim 1, wherein the gap (34) is configured to allow the catheter (60) to deform, or buckle, in the gap, at a movement of the catheter (60) relative to the needle support (10), at an engagement of the catheter (60) by the lock (70).

3. The support structure of any claim 1-2, wherein the support structure (1) comprises a first wall (22) and a second wall (24) that defines, or forms, the gap (34), wherein the first wall (22) is spaced apart from the second wall (24).

4. The support structure of claim 3, wherein the first wall (22) has a first opening (32), that connects the gap (34) to the needle support (10), and is configured to receive and guide the catheter (60) between the needle support (10) and the gap (34).

5. The support structure of claim 3-4, wherein the second wall (24) has a second opening (38), that connects the gap (34) to the lock (10), and is configured to receive and guide the catheter (60) between the gap (34) and the lock (70).

6. The support structure of claims 4 and 5, wherein the first opening (32) defines a first axis (A), and the second opening (38) defines a second axis (B), wherein the first axis (A) and the second axis (B) are parallel.

7. The support structure of claim 3, wherein the first wall (22) is connected to the second wall (24) via a bottom wall (30), or bottom (30), wherein the first wall, the second wall and the bottom wall defines, or forms, the gap (34).

8. The support structure of claim 7, wherein the bottom wall (30) defines a plane, and the gap (34) is configured to allow the catheter to deform, or buckle, parallel to the plane.

9. The support structure of claim 7-8, wherein the bottom wall (30) comprises an indicia (92, 94), mark (92, 94), configured to indicate the amount of deformation of the catheter (60) at the movement of the catheter relative to the needle support (10).

10. The support structure of claim 9, wherein the bottom wall (30) defines a surface, and the indicia (92, 94), is printed, or engraved, or embossed, in the surface.

11. The support structure of claim 3-10, wherein the support structure (1) has a third wall (26) that is connected to the first wall (22) and the second wall (26),
wherein the third wall is convex, or defines an apex (36), and the third wall is configured to contact and pre-bend the catheter (60) that extends through the gap (34).

12. The support structure of claim 6, wherein the support structure (1) comprises a cover (98), that covers the gap (34), wherein the cover (98) is transparent.

13. A system (100) that comprises,
- the support structure (1) of any claim 1-12,
- a hollow needle (40) and a catheter (60),
- wherein the hollow needle (40) is mounted to the needle support (10), and the lock (70), locks the catheter (60) relative to the support structure (1), and
- the catheter (60) extends from the lock (70), through the gap (34), or space (34), enters the hollow needle (40) via the needle support (10), extends through the hollow needle (40) and beyond a needle tip (44), and
- wherein at a movement of the catheter (60) relative to the needle support (10), or the needle tip (44), the catheter (60) deforms, or buckles, in the gap (34), or space (34), and the deformation is visible, or viewable, from outside the support structure (10).

14. The system of claim 13, further comprises a stylet (204) that is received in the catheter (60), wherein the stylet (204) extends from a first end (62) of the catheter (60), or a catheter tip (62), to a second end (64) of the catheter (60), wherein the stylet (204) is configured to be operationally connected to a tissue detection arrangement (200), for detecting, or determining, a type of tissue at the first end (62) of the catheter (60), or a catheter tip (62).

15. The system of claim 13, wherein the catheter (60) is transparent, and has a refractive index that corresponds to, or matches, the refractive index of cerebrospinal fluid.
